# EUROPÄISCHE PATENTSCHRIFT

(11) **EP 4 273 515 B1**
(45) Veröffentlichungstag und Bekanntmachung des Hinweises auf die Patenterteilung: **21.01.2026**
(21) Anmeldenummer: 23171902.2
(22) Anmeldetag: 05.05.2023
(51) Int. Cl.: G01F 15/12, A61L 2/18, B08B 9/032, G01F 13/00, G01N 1/10

(54) **DOSIERVORRICHTUNG, ABFÜLLSYSTEM SOWIE VERFAHREN ZUR REINIGUNG EINER DOSIERVORRICHTUNG**
DOSING DEVICE, FILLING SYSTEM AND METHOD FOR CLEANING A DOSING DEVICE
DISPOSITIF DE DOSAGE, SYSTÈME DE REMPLISSAGE ET PROCÉDÉ DE NETTOYAGE D'UN DISPOSITIF DE DOSAGE

(30) Priorität: 06.05.2022 DE 102022111366
(43) Veröffentlichungstag der Anmeldung: 08.11.2023
(73) Patentinhaber: Ampack GmbH, 86343 Königsbrunn (DE)
(72) Erfinder: Tomschi, Korbinian, 86573 Zahling (DE); Haak, Jürgen, 72820 Sonnenbuehl (DE); Müller, Detlef, 74523 Schwäbisch Hall (DE); Heichele, Bernhard, 86368 Gersthofen (DE)
(74) Vertreter: Daub, Thomas

(56) Entgegenhaltungen:
- EP-A1- 3 872 028
- EP-A2- 3 825 277
- DE-A1- 102014 109 447

## Beschreibung

### Stand der Technik

Aus dem Stand der Technik sind bereits Dosiervorrichtungen bekannt, welche zur regelmäßigen Reinigung mit einer CIP-Anlage verbindbar und/oder koppelbar sind. Ein Reinigungsprozess zur Reinigung der Dosiervorrichtung dauert üblicherweise einige Stunden, währenddessen ein Produktionsprozess unterbrochen oder beendet ist. Bei der Reinigung werden oftmals große Mengen an Energie und/oder Reinigungsmedien in dem Reinigungsprozess verbraucht. Nachtteilig ist im Stand der Technik, dass der Reinigungsprozess in der Regel auf Basis von Zeiterfahrungswerten erfolgt, was häufig zu einer Ressourcenverschwendung führen kann, denn abhängig von den Produkten, welche in dem Produktionsprozess durch die Dosiervorrichtung geführt, und zwar mittels der Dosiervorrichtung abgefüllt werden, werden im Stand der Technik innerhalb des Reinigungsprozesses, und zwar während einzelnen Teilschritten des Reinigungsprozesses zur Reinigung verschiedener Bauteile und/oder Bereiche der Dosiervorrichtung, die verschiedenen Bauteile und/oder Bereiche der Dosiervorrichtung oftmals gründlicher gereinigt als erforderlich. Zwar ist aus dem Stand der Technik bereits bekannt, das Reinigungsmedium vor und nach der Reinigung der Dosiervorrichtung miteinander zu vergleichen, um ein Reinigungsergebnis ermitteln zu können, jedoch erfolgt dies unabhängig und ungeachtet der verschiedenen Bauteile und/oder Bereiche innerhalb der Dosiervorrichtung, sodass das Reinigungsergebnis lediglich Rückschlüsse auf eine gesamte Reinigung der Dosiervorrichtung zulassen kann. Weist das Reinigungsmedium nach der Reinigung beispielsweise noch Produktrückstände auf, ist im Stand der Technik lediglich bekannt, den gesamten Reinigungsprozess weiter durchzuführen und/oder zu verlängern, bis das Reinigungsmedium frei von den Produktrückständen ist, und zwar unabhängig davon, von welchen Bauteilen und/oder Bereichen innerhalb der Dosiervorrichtung die Produktrückstände in das Reinigungsmedium gelangt sind, und gerade welche Bauteile und/oder Bereiche innerhalb der Dosiervorrichtung mehr Reinigung oder keine Reinigung mehr benötigen.

Aus DE 10 2014 109 447 A1 sowie EP 3 825 277 A2 sind bereits Dosiervorrichtungen bekannt, die zumindest eine Abfülleinheit zur Förderung von zumindest flüssigen oder pastösen Produkten, eine Schnittstelle zu einer CIP-Anlage zur Reinigung der Abfülleinheit mittels eines Reinigungsmediums in zumindest einem Reinigungsprozess und eine Sensoreinheit zur Sensierung zumindest einer Verschmutzungskenngröße des Reinigungsmediums in dem Reinigungsprozess umfassen.

Ferner ist aus der EP 3 872 028 A1 ein System und ein Verfahren zur Vorhersage von Schmutz oder Verunreinigungen in einer Trinkwasserleitung und insbesondere ein System und ein Verfahren zur Vorhersage von Schmutz oder Verunreinigungen in einer Leitung, durch die eine Trinkwasserflüssigkeit, wie beispielsweise Bier, fließt, bekannt.

Die Aufgabe der Erfindung besteht insbesondere darin, eine gattungsgemäße Dosiervorrichtung, ein gattungsgemäßes Abfüllsystem und/oder ein gattungsgemäßes Verfahren mit verbesserten Eigenschaften hinsichtlich einer Reinigungseffizienz, insbesondere hinsichtlich einer Erfassung einer Verunreinigung und/oder Verschmutzung des Reinigungsmediums, bereitzustellen. Die Aufgabe wird erfindungsgemäß durch die Merkmale des Anspruchs 1, des Anspruchs 8 bzw. des Anspruchs 9 gelöst, während vorteilhafte Ausgestaltungen und Weiterbildungen der Erfindung den Unteransprüchen entnommen werden können.

### Offenbarung der Erfindung

Die Erfindung geht aus von einer Dosiervorrichtung, insbesondere einer Lebensmitteldosiervorrichtung, mit zumindest einer Abfülleinheit zur Förderung von zumindest flüssigen oder pastösen Produkten, mit einer Schnittstelle zu einer CIP-Anlage zur Reinigung der Abfülleinheit mittels eines Reinigungsmediums in zumindest einem Reinigungsprozess, mit einer Sensoreinheit zur Sensierung zumindest einer Verschmutzungskenngröße des Reinigungsmediums in dem Reinigungsprozess, und mit einer Aufnahmeeinheit zur Aufnahme des Reinigungsmediums, an welcher ein erstes Sensorelement der Sensoreinheit angeordnet ist.

Es wird vorgeschlagen, dass die Abfülleinheit eine Dosiereinheit aufweist und das erste Sensorelement der Sensoreinheit fluidtechnisch hinter einem Produktausgang der Dosiereinheit angeordnet ist, wobei die Aufnahmeeinheit zur Aufnahme des Reinigungsmediums nach einer Reinigung zumindest der Dosiereinheit vorgesehen ist, wobei die Dosiereinheit zumindest eine Dosierpumpe aufweist, welche zur Dosierung der Produkte vorgesehen ist, wobei die Dosierpumpe den Produktausgang aufweist.

Durch eine derartige Ausgestaltung kann eine Reinigungseffizienz gesteigert werden. Ferner kann ein Reinigungsprozess zur Reinigung einer Dosiervorrichtung optimiert und nachhaltiger gestaltet werden. Zudem können Ressourcen, wie beispielsweise Energie und/oder Reinigungsmedien effizient eingesetzt und vorteilhaft eingespart werden. Damit kann wiederum eine Kosten- und/oder eine Produkt- und/oder eine Arbeitseffizienz gesteigert werden. Außerdem kann eine Reinigung effizienter und exakter an eine jeweilige Notwendigkeit der Reinigung einzelner Bauteile und/oder Bereiche der Dosiervorrichtung angepasst werden. Mittels Sensierung zumindest einer Verschmutzungskenngröße des Reinigungsmediums in dem Reinigungsprozess innerhalb der Dosiervorrichtung kann der Reinigungsprozess individuell und/oder flexibel je nach Verunreinigung und/oder Verschmutzung des Reinigungsmediums angepasst werden, um ein bevorzugtes Reinigungsergebnis zu erzielen. Somit kann der Reinigungsprozess je nach Bedarf und/oder Notwendigkeit beispielsweise verkürzt oder verlängert werden, sodass die Reinigungseffizienz hinsichtlich einer zeitlichen Dauer verbessert werden kann. Darüber hinaus kann beispielsweise ein Pausieren einer Produktionsphase verkürzt und damit wiederum eine Produktionseffizienz und ein Komfort gesteigert werden.

Vorzugsweise ist die Dosiervorrichtung Teil eines Abfüllsystems und bildet eine Unterbaugruppe des Abfüllsystems aus. Das Abfüllsystem ist, ohne darauf beschränkt zu sein, zum Abfüllen von flüssigen oder pastösen oder stückigen Produkten vorgesehen. Bei dem Produkt kann es sich beispielsweise um ein pharmazeutisches Produkt, ein Bedarfsgut und/oder Beauty- und/oder Körperpflegeprodukt und/oder um vorteilhaft ein Lebensmittel handeln, wie beispielsweise Soßen, Milchprodukte, insbesondere Joghurts, Brotaufstriche, Pasten, Fertiggerichte, Süßwaren, Cremes, und/oder Shampoos oder dergleichen. Insbesondere handelt es sich bei dem Abfüllsystem um ein Lebensmittelabfüllsystem. Das Abfüllsystem kann eine Vielzahl an Einheiten und/oder Elementen und/oder Vorrichtungen aufweisen, welche zu einer Be- und/oder Verarbeitung und/oder Dosierung und/oder Formung und/oder Verpackung und/oder Verschließung und/oder Lagerung von Produkten, insbesondere Lebensmitteln, verwendet werden könnte. Bevorzugt weist das Abfüllsystem die CIP-Anlage auf. Insbesondere handelt es sich bei der CIP-Anlage um eine einem Fachmann bekannte Cleaning-inplace Anlage. Die CIP-Anlage kann eine Vielzahl an Einheiten und/oder Elementen und/oder Vorrichtungen aufweisen, welche dazu vorgesehen sind und/oder dazu beitragen, das Reinigungsmedium aufzunehmen, zu speichern, zu lagern, zu pumpen, zu leiten, zu analysieren, zu sensieren, aufzufangen und/oder zu verarbeiten, insbesondere weiter zu verarbeiten.

Die Dosiervorrichtung ist zur Dosierung zumindest von flüssigen oder pastösen Produkten vorgesehen. Alternativ und/oder zusätzlich könnte die Dosiervorrichtung auch zur Dosierung von stückigen Produkten vorgesehen sein. Denkbar wäre, dass die Dosiervorrichtung beispielsweise eine hydraulische Dosiervorrichtung, insbesondere für ein Nutz- und/oder Kraftfahrzeug, ist. Vorzugsweise handelt es sich bei der Dosiervorrichtung um eine Lebensmitteldosiervorrichtung. Insbesondere fördert die Abfülleinheit die Produkte von zumindest einem Eingang der Dosiervorrichtung zu zumindest einem Ausgang der Dosiervorrichtung. Bevorzugt umfasst die Abfülleinheit eine Vielzahl an Einheiten und/oder Elementen und/oder Vorrichtungen, welche zur Abfüllung, und zwar zur Dosierung der Produkte verwendbar, insbesondere dazu vorgesehen und/oder bereitgestellt und/oder geeignet, sind.

Die Schnittstelle der Dosiervorrichtung zu der CIP-Anlage kann beispielsweise aus zumindest einer Fluidleitung des Abfüllsystems bestehen, mittels welcher zumindest die Abfülleinheit mit der CIP-Anlage verbindbar und/oder an die CIP-Anlage anschließbar ist. Bevorzugt weist das Abfüllsystem zumindest eine Fluideingangsleitung, insbesondere eine Reinigungsmitteleingangsleitung, und zumindest eine Fluidausgangsleitung, insbesondere eine Reinigungsmittelausgangsleitung, auf, um die Abfülleinheit mit der CIP-Anlage zu verbinden. In dem Reinigungsprozess kann das Reinigungsmedium von der CIP-Anlage durch die Fluideingangsleitung zu der Abfülleinheit geführt und/oder geleitet werden. Ferner kann in dem Reinigungsprozess das Reinigungsmedium von der Abfülleinheit durch die Fluidausgangsleitung zu der CIP-Anlage geführt und/oder geleitet werden. Denkbar wäre auch, dass das Abfüllsystem eine Vielzahl von Fluideingangsund/oder Fluidausgangsleitungen, und zwar zumindest zwei Fluideingangsleitungen und/oder zumindest zwei Fluidausgangsleitungen aufweist. Ferner könnte/könnten die Fluidausgangsleitung und/oder die Fluideingangsleitung auch Teil der CIP-Anlage und/oder der Dosiervorrichtung sein. Bevorzugt weist die Dosiervorrichtung zumindest teilweise eine Fluidleitung, insbesondere zumindest die Fluidausgangsleitung und/oder die Fluideingangsleitung, zur Verbindung mit der CIP-Anlage auf. Vorteilhaft unabhängig davon durch welche Elemente und/oder Einheiten und/oder Bereiche und/oder Bauteile der Abfülleinheit das Reinigungsmedium geleitet oder geführt wird, ist das Reinigungsmedium stets durch die allgemeine Fluideingangsleitung zur Abfülleinheit leitbar und/oder führbar sowie durch die allgemeine Fluidausgangsleitung von der Abfülleinheit wieder ableitbar und/oder abführbar. In dem Reinigungsprozess könnte die Dosiervorrichtung zumindest teilweise automatisch und/oder manuell, oder vollständig automatisch oder manuell, mit der CIP-Anlage verbunden werden.

Unter dem Reinigungsprozess soll vorliegend ein Prozess verstanden werden, in welchem zumindest die Abfülleinheit zumindest teilweise und bevorzugt zumindest zu einem Großteil gereinigt wird. Insbesondere werden in dem Reinigungsprozess zumindest jene Teile der Abfülleinheit gereinigt, welche mit dem Produkt bei Abfüllung des Produkts in Kontakt sind und/oder in welchen sich das Produkt zumindest zeitweise bei Abfüllung befindet und/oder dort gelagert ist. Vorzugsweise ist der Reinigungsprozess dazu vorgesehen, Produktreste aus zumindest Teilen der Abfülleinheit, bevorzugt der gesamten Abfülleinheit, zu entfernen, insbesondere vollständig zu entfernen. Der Reinigungsprozess kann sowohl ein sogenanntes Zwischenspülen, beispielsweise bei einem Produktwechsel innerhalb einer Produktionsphase, als auch ein Endreinigungsprozess, beispielsweise am Ende der Produktionsphase, sein. Der Produktwechsel kann einen Wechsel von Produkten innerhalb derselben Produktkategorie umfassen, beispielsweise von einem Vanillejoghurt, welcher ein erstes Produkt bildet, zu einem Schokojoghurt, welcher ein zweites Produkt bildet, oder andersrum. Vorzugsweise erfolgt zwischen dem jeweiligen Abfüllen der verschiedenen Produkte der als Zwischenspülen benannte Reinigungsprozess, um vorteilhaft Produktrückstände des ersten Produkts vor einem Abfüllen des zweiten Produkts zu entfernen. Insbesondere erfolgt der Reinigungsprozess, bevorzugt das Zwischenspülen, zwischen zwei Abfüllprozessen unterschiedlicher Produkte, und zwar während der Produktionsphase. Möglicherweise wäre denkbar, dass der Reinigungsprozess in der Produktionsphase während eines Betriebs der Abfülleinheit erfolgt. Vorteilhaft erfolgt der Reinigungsprozess, insbesondere das Zwischenspülen, in der Produktionsphase während einer Betriebspause der Abfülleinheit. Bei dem Zwischenspülen kann sich das Reinigungsmedium zumindest zu einem Großteil und vorteilhaft vollständig aus Wasser zusammensetzen. Aufgrund des Produktwechsels in derselben Produktkategorie kann auf zusätzliche Stoffe und/oder Chemikalien im Reinigungsmedium beim Zwischenspülen verzichtet werden. Damit kann ein Produktwechsel schneller und effizienter gestaltet werden. Ferner können Ressourcen, wie Energie und/oder Reinigungsmedien, beispielsweise Wasser, effizient eingesetzt und vorteilhaft eingespart werden.

Besonders bevorzugt werden bei dem Endreinigungsprozess Chemikalien und/oder zusätzliche Stoffe zur Reinigung verwendet, sodass das Reinigungsmedium jene Chemikalien und/oder zusätzlichen Stoffe aufweist. Der Reinigungsprozess, und zwar der Endreinigungsprozess kann am Ende der Produktionsphase, und zwar am Ende der Abfüllung von bevorzugt mehreren und unterschiedlichen Produkten, wie beispielsweise dem ersten Produkt und/oder zumindest dem zweiten Produkt, erfolgen. Unter dem Ausdruck "zu einem Großteil" sollen vorliegend beispielsweise zumindest 60 %, vorteilhaft zumindest 70 %, vorzugsweise zumindest 80 %, besonders bevorzugt zumindest 90 % und besonders vorteilhaft höchstens 99 % eines Volumen- und/oder Massenanteils verstanden werden.

Die Sensoreinheit ist dazu vorgesehen, zumindest die Verschmutzungskenngröße des Reinigungsmediums in dem Reinigungsprozess zu sensieren und/oder zu erfassen und/oder zu erkennen. Die Sensoreinheit könnte genau ein Sensorelement aufweisen. Das Sensorelement könnte die Verschmutzungskenngröße des Reinigungsmediums erfassen und/oder sensieren. Bevorzugt umfasst die Sensoreinheit mehrere Sensorelemente zur Sensierung des Reinigungsmediums an einzelnen und unterschiedlichen Stellen innerhalb der Abfülleinheit. Die einzelnen Sensorelemente der Sensoreinheit können jeweils einzelne Kenngrößen, beispielsweise Verschmutzungskenngrößen und/oder Referenzausgangskenngrößen für bevorzugte Vergleiche mit den Verschmutzungskenngrößen, des Reinigungsmediums sensieren und/oder erfassen, mittels welchen die Verschmutzung und/oder Verunreinigung des Reinigungsmediums ermittelbar ist. Bevorzugt kann mittels Sensierung und/oder Erfassung von Kenngrößen des Reinigungsmediums durch mehrere Sensorelemente, welche an unterschiedlichen Stellen und/oder Positionen innerhalb der Abfülleinheit angeordnet sind, eine präzise Aussage über die Verschmutzung und/oder Verunreinigung des Reinigungsmediums, insbesondere an den jeweiligen Stellen innerhalb der Abfülleinheit, getroffen werden. Vorteilhaft weist die Sensoreinheit zumindest ein optisches Sensorelement zur Sensierung der Kenngröße, insbesondere zumindest der Verschmutzungskenngröße, auf.

Insbesondere charakterisiert und/oder definiert die Verschmutzungskenngröße die Verschmutzung und/oder Verunreinigung des Reinigungsmediums, und zwar einen Grad der Verschmutzung und/oder Verunreinigung. Beispielsweise wenn die Verschmutzungskenngröße einen hohen Wert aufweist, kann das Reinigungsmedium stark verschmutzt sein. Weist die Verschmutzungskenngröße einen, insbesondere zu dem hohen Wert vergleichbaren und/oder in Relation setzbaren niedrigen Wert auf, kann das Reinigungsmedium verschmutzungsarm und zumindest zu einem Großteil oder bevorzugt vollständig frei von Verschmutzungen sein. Die Verschmutzungskenngröße kann beispielsweise als absoluter Wert mit bereits bekannten Werten, insbesondere Vergleichswerten, verglichen werden, um damit die Verschmutzung und/oder Verunreinigung des Reinigungsmediums, und zwar einen Grad der Verschmutzung und/oder Verunreinigung zu bestimmen und/oder zu ermitteln. Alternativ oder zusätzlich kann zur Ermittlung der Verschmutzung und/oder Verunreinigung des Reinigungsmediums die Verschmutzungskenngröße in Relation mit einer ebenfalls von der Dosiervorrichtung sensierten weitere Kenngröße gesetzt werden.

Die Dosiervorrichtung kann eine Steuereinheit aufweisen, welche dazu vorgesehen ist, den Reinigungsprozess basierend auf einem Analyseergebnis der Verschmutzungskenngröße zu steuern und/oder zu regeln. Unter einer "Steuereinheit" soll eine elektronische Einheit verstanden werden, die vorteilhaft dazu vorgesehen ist, zumindest den Reinigungsprozess zu steuern und/oder zu regeln. Vorzugsweise umfasst die Steuereinheit eine Recheneinheit und insbesondere zusätzlich zur Recheneinheit eine Speichereinheit mit einem darin gespeicherten Steuer- und/oder Regelprogramm, das dazu vorgesehen ist, von der Recheneinheit ausgeführt zu werden.

Insbesondere ist die Steuereinheit zumindest zu einem Starten und/oder Beginnen und/oder Pausieren und/oder Aussetzen und/oder Beenden des Reinigungsprozesses vorgesehen. Ferner kann die Steuereinheit eine zeitliche Dauer und/oder einen zeitlichen Verlauf des Reinigungsprozesses steuern und/oder regeln. Basierend auf dem Analyseergebnis der Verschmutzungskenngröße kann die Steuereinheit den Reinigungsprozess beispielsweise beenden und/oder verkürzen und/oder verlängern und/oder möglicherweise pausieren. Damit kann eine Steuerung des Reinigungsprozesses individuell und flexibel angepasst und vorteilhaft Ressourcen, und zwar das Reinigungsmedium effizient genutzt werden.

Bevorzugt ist die Steuereinheit zusätzlich zur Steuerung des Reinigungsprozesses dazu vorgesehen, die Produktionsphase, und zwar den Betrieb und das Abfüllen von Produkten mittels der Abfülleinheit zu steuern und/oder zu regeln. Die Steuereinheit kann das Abfüllen von Produkten beenden und den Reinigungsprozess starten. Beispielsweise kann die Steuereinheit das Abfüllen des ersten Produkts beenden und das Zwischenspülen starten. Nach Beenden des Zwischenspülens könnte die Steuereinheit das Abfüllen des zweiten Produkts starten. Alternativ könnte die Steuereinheit die gesamte Produktionsphase beenden und den Reinigungsprozess, und zwar den Endreinigungsprozess zumindest starten und steuern und/oder regeln.

Darüber hinaus geht die Erfindung aus von einem Verfahren zur Reinigung einer Dosiervorrichtung, insbesondere der zuvor genannten Dosiervorrichtung, mit einer Abfülleinheit zur Förderung von zumindest flüssigen oder pastösen Produkten und mit einer Schnittstelle zu einer CIP-Anlage zur Reinigung der Abfülleinheit mittels eines Reinigungsmediums in zumindest einem Reinigungsprozess, wobei in dem Reinigungsprozess zumindest eine Verschmutzungskenngröße des Reinigungsmediums sensiert wird, wobei die Verschmutzungskenngröße des Reinigungsmediums durch ein erstes an einer Aufnahmeeinheit der Dosiervorrichtung angeordnetes, insbesondere das bereits zuvor gennannte, Sensorelement einer, insbesondere der bereits zuvor gennannten, Sensoreinheit der Dosiervorrichtung sensiert und/oder erfasst wird. Es wird vorgeschlagen, dass die Aufnahmeeinheit das Reinigungsmedium nach einer Reinigung zumindest einer, insbesondere der bereits zuvor genannten, zumindest eine Dosierpumpe zu einer Dosierung von Produkten umfassenden Dosiereinheit der Dosiervorrichtung aufnimmt, wobei in einem ersten Reinigungsschritt das Reinigungsmedium durch einen Reinigungsmittelzulauf einer Produkttankeinheit der Abfülleinheit zu einem Produktausgang der Dosiereinheit geleitet wird, wobei die Dosierpumpe den Produktausgang aufweist. Durch ein derartiges Reinigungsverfahren kann eine Reinigungseffizienz optimiert und Ressourcen, wie beispielsweise Energie und/oder Reinigungsmedien effizienter eingesetzt und vorteilhaft eingespart werden. Zudem kann eine Reinigungseffizienz hinsichtlich einer Reinigung einer Produkttankeinheit, und zwar zumindest einer Produktzufuhrleitung der Produkttankeinheit sowie einer Dosiereinheit einer Abfülleinheit einer Dosiervorrichtung optimiert werden. Zudem kann eine zeitliche Dauer eines Reinigungsprozesses, bevorzugt zumindest eines ersten Reinigungsschritts des Reinigungsprozesses, individuell und/oder flexibel je nach Verunreinigung und/oder Verschmutzung des Reinigungsmediums angepasst werden. Damit können wiederum Ressourcen, wie beispielsweise Energie und/oder Reinigungsmedien effizienter eingesetzt werden.

Insbesondere handelt es sich bei der genannten Abfülleinheit um die bereits genannte Abfülleinheit, bei der CIP-Anlage um die bereits genannte CIP-Anlage, bei dem Reinigungsprozess um den bereits genannten Reinigungsprozess, bei dem Reinigungsmedium um das bereits genannte Reinigungsmedium und bei der Verschmutzungskenngröße um die bereits genannte Verschmutzungskenngröße. Vorteilhaft wird der Reinigungsprozess basierend auf dem Analyseergebnis der Verschmutzungskenngröße gesteuert und/oder geregelt. Das Verfahren zur Reinigung der Dosiervorrichtung kann mehrere Verfahrensschritte aufweisen. Bevorzugt umfasst das Verfahren zumindest einen ersten Reinigungsschritt und einen zweiten Reinigungsschritt. Ferner kann das Verfahren noch weitere Reinigungsschritte, wie beispielsweise einen dritten Reinigungsschritt und/oder einen vierten Reinigungsschritt umfassen. Der erste Reinigungsschritt kann, aber ohne darauf beschränkt zu sein, hinsichtlich eines zeitlichen Verlaufs des Verfahrens vor dem zweiten, dem dritten und/oder zumindest dem vierten Reinigungsschritt erfolgen. Denkbar wäre auch, dass die Reihenfolge der Reinigungsschritte vertauschbar und/oder abänderbar ist und/oder einzelne Reinigungsschritte ausgelassen und/oder weggelassen werden können.

Im vorliegenden Dokument dienen Zahlwörter, wie beispielsweise "erste/r/s" und "zweite/r/s", welche bestimmten Begriffen vorangestellt sind, lediglich zu einer Unterscheidung von Objekten und/oder einer Zuordnung zwischen Objekten untereinander und implizieren keine vorhandene Gesamtanzahl und/oder Rangfolge der Objekte. Insbesondere impliziert ein "zweites Objekt" nicht zwangsläufig ein Vorhandensein eines "ersten Objekts". Ferner soll unter "vorgesehen" hier und im Folgenden speziell programmiert, ausgelegt und/oder ausgestattet verstanden werden. Darunter, dass ein Objekt zu einer bestimmten Funktion vorgesehen ist, soll verstanden werden, dass das Objekt diese bestimmte Funktion in zumindest einem Anwendungs- und/oder Betriebszustand erfüllt und/oder ausführt.

Bei der Verschmutzung des Reinigungsmediums kann es sich beispielsweise um Produktrückstände handeln. Insbesondere tragen die Produktrückstände zur Verschmutzung des Reinigungsmediums bei. Ferner wird vorgeschlagen, dass die Sensoreinheit dazu vorgesehen ist, Rückstände von Produkten in dem Reinigungsmedium zu sensieren. Hierdurch kann eine Reinigungseffizienz weiter optimiert und die Reinigung effizienter und exakter an eine jeweilige Notwendigkeit der Reinigung einzelner Bauteile und/oder Bereiche einer Dosiervorrichtung angepasst werden. Zudem kann festgestellt und/oder ermittelt werden, von welchen Bauteilen und/oder Bereichen innerhalb der Dosiervorrichtung die Produktrückstände in das Reinigungsmedium gelangt sind und damit wiederum der Reinigungsprozess individuell und/oder flexibel angepasst werden.

Beispielsweise kann die Sensoreinheit in dem Reinigungsprozess, insbesondere während des Zwischenspülens, erfassen und/oder sensieren, ob Rückstände des ersten Produkts in dem Reinigungsmedium enthalten sind. Erst wenn das Reinigungsmedium vorteilhaft frei von Produktrückständen, beispielsweise des ersten Produkts ist, kann die Steuereinheit den Reinigungsprozess beenden. Hinsichtlich des Zwischenspülens kann die Steuereinheit, nachdem das Reinigungsmedium frei von Produktrückständen des ersten Produkts ist, das Abfüllen des zweiten Produkts starten und/oder aktivieren und/oder steuern und/oder regeln. Insbesondere umfasst die Verschmutzungskenngröße eine Information und/oder einen Wert, welche beschreibt und/oder charakterisiert, ob Produktrückstände in dem Reinigungsmedium enthalten sind. Bevorzugt umfasst die Verschmutzungskenngröße zusätzlich eine Information und/oder einen Wert, welcher beschreibt und/oder charakterisiert, wie hoch das Maß an Produktrückständen in dem Reinigungsmedium ist. Insbesondere je mehr Produktrückstände in dem Reinigungsmedium enthalten sind, desto größer ist der Grad der Verschmutzung und/oder Verunreinigung des Reinigungsmediums. Besonders bevorzugt kann die Steuereinheit das Maß an Produktrückständen in dem Reinigungsmedium basierend auf zumindest der Verschmutzungskenngröße sowie den Grad der Verschmutzung und/oder Verunreinigung des Reinigungsmediums ermitteln und/oder analysieren und vorteilhaft basierend auf dem Analyseergebnis den Reinigungsprozess steuern und/oder regeln.

Um eine Reinigungseffizienz sowie eine Erfassung und/oder Ermittlung und/oder Analyse eines Reinigungsmediums weiter zu verbessern, wird vorgeschlagen, dass die Sensoreinheit dazu vorgesehen ist, einen Trübungsgrad des Reinigungsmediums zu sensieren. Zudem kann damit eine Reinigung weiter optimiert und der Reinigungsprozess individuell und/oder flexibel je nach Verunreinigung und/oder Verschmutzung des Reinigungsmediums angepasst werden, um ein bevorzugtes Reinigungsergebnis zu erzielen.

Die Sensoreinheit kann einen Trübungssensor umfassen. Vorteilhaft weist eines der Sensorelemente den Trübungssensor auf oder ist als solcher ausgebildet. Insbesondere ist die Verschmutzungskenngröße eine Kenngröße des Trübungsgrads, welche eine Trübung des Reinigungsmediums beschreibt und/oder charakterisiert. Insbesondere je höher der Trübungsgrad des Reinigungsmediums ist, desto stärker ist die Trübung und damit wiederum der Grad der Verschmutzung und/oder Verunreinigung des Reinigungsmediums. Besonders bevorzugt kann die Steuereinheit die Trübung des Reinigungsmediums basierend auf dem Trübungsgrad sowie den Grad der Verschmutzung und/oder Verunreinigung des Reinigungsmediums ermitteln und/oder analysieren und vorteilhaft basierend auf dem Analyseergebnis den Reinigungsprozess steuern und/oder regeln. Zusätzlich kann die Sensoreinheit zur Erfassung der Verschmutzung und/oder Verunreinigung des Reinigungsmediums weitere Kenngrößen, wie beispielsweise eine Temperatur, einen Volumenstrom, einen Leitwert oder dergleichen sensieren.

Erfindungsgemäß umfasst die Abfülleinheit die Dosiereinheit, wobei das erste Sensorelement der Sensoreinheit fluidtechnisch hinter dem Produktausgang der Dosiereinheit angeordnet ist. Dadurch kann die Reinigungseffizienz sowie eine Erfassung eines Reinigungsergebnisses innerhalb eines Reinigungsprozesses, und zwar fluidtechnisch hinter einem Produktausgang einer Dosiereinheit einer Abfülleinheit einer Dosiervorrichtung verbessert werden. Zudem kann basierend auf einem Ergebnis einer Verunreinigung und/oder Verschmutzung des Reinigungsmediums fluidtechnisch hinter dem Produktausgang der Reinigungsprozess individuell und/oder flexibel angepasst, beispielsweise eine zeitliche Dauer des Reinigungsprozesses verlängert und/oder verkürzt werden.

Insbesondere ist die Dosiereinheit zur Förderung von zumindest flüssigen oder pastösen Produkten vorgesehen. Die Dosiereinheit weist zumindest eine Dosierpumpe auf, welche zur Dosierung der Produkte vorgesehen ist. Bevorzugt umfasst die Dosiereinheit eine Vielzahl, bevorzugt ein ganzzahliges Vielfaches, an Dosierpumpen, welche vorteilhaft nebeneinander beispielsweise in einer insbesondere einzelnen Reihe oder matrix-förmig angeordnet sein können. Bevorzugt weist die Dosiereinheit eine gerade Anzahl an Dosierpumpen auf. Es ist jedoch auch denkbar, dass die Dosiereinheit eine ungerade Anzahl an Dosierpumpen umfasst, wie beispielsweise drei Dosierpumpen oder fünf Dosierpumpen. Die Dosierpumpen sind vorzugsweise einzeln über, insbesondere individuelle, Zuführleitungen der Dosiereinheit mit einer Produkttankeinheit der Dosiervorrichtung, insbesondere der Abfülleinheit, verbunden. Die Produkttankeinheit umfasst insbesondere zumindest ein Tankelement, in welchem zumindest die oder das flüssige/n oder pastöse/n Produkt/e anordenbar ist/sind. Insbesondere ist die Produkttankeinheit zur Aufnahme und/oder Lagerung, insbesondere Zwischenlagerung, des Produkts oder der Produkte vorgesehen. Das Tankelement ist bevorzugt als Tank ausgebildet und kann alternativ auch beispielsweise als Beutel oder Trichter ausgebildet sein. Denkbar ist auch, dass die Produkttankeinheit eine Vielzahl an Tankelementen umfasst, wobei in den einzelnen Tankelementen unterschiedliche Produkte anordenbar sind, welche mittels der Dosiereinheit in Produktbehälter förderbar und/oder dosierbar sind, um mehrschichte Produkte oder Produkte mit unterschiedlichen Bestandteilen zu ermöglichen, wie beispielsweise einen Pudding mit Sahnehaube, der in einem Becher abgefüllt ist oder dergleichen. Insbesondere abhängig von dem Produkt und/oder den Produkten kann der Produktbehälter zumindest teilweise oder zumindest zu einem Großteil, insbesondere vollständig, aus einem Metall, beispielsweise Edelstahl, einem Mineral, beispielsweise Glas und/oder Papier und/oder einem faserbasierten Stoff und/oder Holz und/oder Keramik, einem Kunststoff und/oder einem Verbundmaterial bestehen und/oder hergestellt sein.

Es ist auch denkbar, dass jeder Dosierpumpe ein einzelnes Tankelement der Produkttankeinheit zugeordnet ist. Bevorzugt sind die Dosierpumpen alle einzeln, insbesondere individuell, über Zuführleitungen mit demselben Tankelement verbunden. Die Zuführleitungen zu den Dosierpumpen sind vorzugsweise mittels einer form- und/oder kraftschlüssigen Verbindung, wie beispielsweise mittels einer Schraubverbindung, einer Klemmverbindung, einer Rastverbindung oder dergleichen, an dem Tankelement fixiert, insbesondere einzeln an dem Tankelement fixiert. Vorteilhaft sind die Zuführleitungen vorzugsweise einzeln reversibel montierbar/demontierbar. Weitere, einem Fachmann als sinnvoll erscheinende Ausgestaltungen und/oder Aufteilungen der Produkttankeinheit und der Dosiereinheit sind ebenfalls denkbar.

Die Dosierpumpe weist den Produktausgang auf. Durch den Produktausgang kann das Produkt in den Produktbehälter abgefüllt und/oder dosiert und/oder befördert und/oder eingeführt und/oder eingebracht werden. Bei dem ersten Sensorelement handelt es sich insbesondere um ein Sensorelement der Sensoreinheit zur Sensierung zumindest einer Verschmutzungskenngröße des Reinigungsmediums fluidtechnisch hinter dem Produktausgang. Nachdem das Reinigungsmedium durch den Produktausgang geflossen ist und/oder den Produktausgang passiert hat, kann das erste Sensorelement die Verschmutzungskenngröße des Reinigungsmediums sensieren und/oder erfassen.

Insbesondere handelt es sich bei der Produkttankeinheit um die bereits genannte Produkttankeinheit, bei dem Produktausgang um den bereits genannten Produktausgang und bei der Dosiereinheit um die bereits genannte Dosiereinheit. In zumindest dem ersten Reinigungsschritt kann das Reinigungsmedium von der CIP-Anlage durch die Fluideingangsleitung zu der Abfülleinheit geleitet werden. Bevorzugt wird in dem ersten Reinigungsschritt die Fluideingangsleitung mit dem Reinigungsmittelzulauf der Produkttankeinheit verbunden, um das Reinigungsmedium von der CIP-Anlage zu der Produkttankeinheit zu leiten und/oder zu führen. Insbesondere ist der Reinigungsmittelzulauf mit dem Tankelement verbunden und/oder an dem Tankelement angeordnet. Der Reinigungsmittelzulauf könnte beispielsweise zumindest eine Leitung zur Leitung und/oder Führung des Reinigungsmediums von der Fluideingangsleitung zu zumindest dem Tankelement aufweisen. Vorzugsweise umfasst der Reinigungsmittelzulauf zumindest eine bevorzugt rotierbare und/oder bewegbare Sprühkugel. Alternativ könnte die Sprühkugel auch als stationäre Sprühkugel ausgebildet sein. Insbesondere ist die Sprühkugel auch als Sprühkopf bezeichnenbar. Zumindest die Sprühkugel kann innerhalb des Tankelements angeordnet sein und sich in dem ersten Reinigungsschritt bevorzugt innerhalb des Tankelements bewegen, beispielsweise zumindest rotieren. Die Sprühkugel könnte sich in Höhenrichtung, in Breitenrichtung und/oder in Tiefenrichtung innerhalb des Tankelements bewegen, um das Tankelement zu reinigen. Denkbar wäre auch, dass der Reinigungsmittelzulauf mehrere Sprühkugeln, beispielsweise zwei, vier oder sieben Sprühkugeln, aufweist.

Insbesondere wird in dem ersten Reinigungsschritt das Reinigungsmedium von dem Reinigungsmittelzulauf durch das Tankelement zur Reinigung des Tankelements, und anschließend zur Reinigung der Dosiereinheit zu der Dosiereinheit, und zwar durch die Zuführleitungen von dem Tankelement zu den Dosierpumpen, ferner zu dem Produktausgang der Dosiereinheit, geleitet und/oder geführt. Das Reinigungsmedium, welches am Produktausgang austritt, wird in zumindest dem ersten Reinigungsschritt vorteilhaft anschließend durch die Fluidausgangsleitung zurück zur CIP-Anlage geführt. Bevorzugt wird in zumindest dem ersten Reinigungsschritt zumindest eine Verschmutzungskenngröße mittels des ersten Sensorelements sensiert und/oder erfasst. Die Steuereinheit kann in dem ersten Reinigungsschritt den Grad der Verschmutzung und/oder Verunreinigung des Reinigungsmediums basierend auf zumindest der Verschmutzungskenngröße ermitteln und/oder analysieren. Vorteilhaft wird der erste Reinigungsschritt, und zwar zumindest eine zeitliche Dauer des ersten Reinigungsschritts mittels der Steuereinheit basierend auf dem Analyseergebnis gesteuert und/oder geregelt.

Es wäre denkbar, dass das erste Sensorelement unmittelbar fluidtechnisch hinter dem Produktausgang der Dosiereinheit, insbesondere der Dosierpumpe, angeordnet ist. Weist die Dosiereinheit mehrere Dosierpumpen auf, könnte möglicherweise an jedem einzelnen Produktausgang der Dosierpumpen ein erstes Sensorelement der Sensoreinheit angeordnet sein, wobei die Sensoreinheit eine Vielzahl von ersten Sensorelementen aufweist. Die Sensoreinheit könnte genauso viele erste Sensorelemente aufweisen, wie die Dosiereinheit Dosierpumpen aufweist. Alternativ könnte das erste Sensorelement auch mittelbar, und zwar beabstandet fluidtechnisch hinter dem Produktausgang angeordnet sein. Vorzugsweise umfasst die Sensoreinheit genau ein erstes Sensorelement, welches insbesondere fluidtechnisch hinter allen einzelnen Produktausgängen der Dosierpumpen angeordnet ist. Bevorzugt ist das erste Sensorelement zwar fluidtechnisch hinter dem Produktausgang der Dosiereinheit, aber fluidtechnisch vor der Fluidausgangsleitung angeordnet. Alternativ und/oder zusätzlich könnte jedoch auch das erste Sensorelement an der Fluidausgangsleitung angeordnet sein, insbesondere die Fluidausgangsleitung das erste Sensorelement oder ein zusätzliches erstes Sensorelement aufweisen.

Erfindungsgemäß umfasst die Dosiervorrichtung die Aufnahmeeinheit zur Aufnahme des Reinigungsmediums nach einer Reinigung zumindest der Dosiereinheit, an welcher das erste Sensorelement angeordnet ist. Damit kann eine Konstruktion hinsichtlich einer Aufnahme und/oder einem Auffangen eines Reinigungsmediums zumindest nach Reinigung einer Dosiereinheit in einem Reinigungsprozess verbessert und eine Reinigungseffizienz bezüglich einer Erfassung einer Verunreinigung und/oder Verschmutzung des Reinigungsmediums zumindest nach Reinigung der Dosiereinheit optimiert werden.

Insbesondere ist die Aufnahmeeinheit fluidtechnisch hinter dem Produktausgang der Dosiereinheit angeordnet. Die Aufnahmeeinheit kann beispielsweise einen Beutel, einen Trichter, einen Tank, eine Schale, eine Schüssel oder ein Auffangbecken aufweisen oder als solche/solches ausgebildet sein. Vorzugsweise handelt es sich bei der Aufnahmeeinheit um eine sogenannte Spülplatte. Die Aufnahmeeinheit kann das Reinigungsmedium nach Durchlauf durch den Produktausgang und somit nach Reinigung der Dosiereinheit, insbesondere zumindest der Dosierpumpe aufnehmen. Das Reinigungsmedium, welches am Produktausgang austritt, wird in zumindest dem ersten Reinigungsschritt vorteilhaft anschließend von der Aufnahmeeinheit aufgenommen und durch die Fluidausgangsleitung zurück zur CIP-Anlage geführt.

Außerdem wird vorgeschlagen, dass in dem Reinigungsprozess die Aufnahmeeinheit in Höhenrichtung betrachtet unterhalb der Dosiereinheit anordenbar ist. Dadurch kann eine Effizienz, hinsichtlich einer Produkt- und/oder eine Arbeits- und/oder Konstruktions- und/oder Reinigungseffizienz weiter gesteigert werden.

Die Aufnahmeeinheit könnte in Höhenrichtung betrachtet dauerhaft, insbesondere unbewegbar, unterhalb der Dosiereinheit, insbesondere den Dosierpumpen, angeordnet sein. Vorteilhaft ist die Aufnahmeeinheit zumindest in Breitenrichtung und/oder in Tiefenrichtung bewegbar gelagert. Zusätzlich und/oder alternativ könnte die Aufnahmeeinheit auch in Höhenrichtung, insbesondere vertikal, bewegbar gelagert sein. Beispielsweise kann sich die Aufnahmeeinheit vertikal in Relation zu der Dosiereinheit bewegen. Die Aufnahmeeinheit kann in dem Reinigungsprozess zur Aufnahme des Reinigungsmediums in Höhenrichtung betrachtet unterhalb der Dosiereinheit, insbesondere den Dosierpumpen, anordenbar sein. Bevorzugt wird in dem ersten Reinigungsschritt die Aufnahmeeinheit in Höhenrichtung betrachtet unterhalb der Dosiereinheit, insbesondere den Dosierpumpen, angeordnet. Zur Anordnung der Aufnahmeeinheit unterhalb der Dosierpumpen, könnten die Dosierpumpen in Höhenrichtung verschiebbar gelagert sein. In dem Reinigungsprozess, insbesondere in zumindest dem ersten Reinigungsschritt, können die Dosierpumpen in Höhenrichtung betrachtet verschoben werden, um die Aufnahmeeinheit unterhalb der Dosierpumpen anzuordnen und/oder zu platzieren. Alternativ wäre auch denkbar, dass die Dosierpumpen unbewegbar und/oder fest angeordnet sind und die Aufnahmeeinheit in Höhenrichtung insbesondere relativ zu den Dosierpumpen verschiebbar gelagert ist.

Insbesondere wird das Reinigungsmedium in dem ersten Reinigungsschritt durch die Dosierpumpen zu der Aufnahmeeinheit geleitet und/oder geführt. Die Aufnahmeeinheit kann mittelbar oder unmittelbar mit der zuvor genannten Fluidausgangsleitung verbunden sein. Besonders bevorzugt ist an der Aufnahmeeinheit die bereits zuvor genannte Fluidausgangsleitung zumindest teilweise angeordnet, insbesondere zur Leitung und/oder Führung des Reinigungsmediums zurück zur CIP-Anlage. Die Fluidausgangsleitung kann in dem Reinigungsprozess neben der Verbindung mit der Aufnahmeeinheit noch weitere, insbesondere gleichzeitige, Verbindungen mit anderen Bauteilen und/oder Elementen und/oder Einheiten der Abfülleinheit aufweisen.

Hinsichtlich des Verfahrens zur Reinigung der Dosiervorrichtung wird außerdem vorgeschlagen, dass in einem zweiten Reinigungsschritt das Reinigungsmedium durch eine Produktzufuhrleitung der Produkttankeinheit zu dem Produktausgang der Dosiereinheit geleitet wird. Dadurch kann eine Reinigungseffizienz hinsichtlich einer Reinigung einer Produkttankeinheit, und zwar zumindest einer Produktzufuhrleitung der Produkttankeinheit sowie einer Dosiereinheit einer Abfülleinheit einer Dosiervorrichtung optimiert werden. Zudem kann eine zeitliche Dauer eines Reinigungsprozesses, bevorzugt zumindest eines zweiten Reinigungsschritts des Reinigungsprozesses, individuell und/oder flexibel je nach Verunreinigung und/oder Verschmutzung des Reinigungsmediums angepasst werden. Damit können wiederum Ressourcen, wie beispielsweise Energie und/oder Reinigungsmedien effizienter eingesetzt werden.

Hinsichtlich des zeitlichen Verlaufs des Verfahrens kann der zweite Reinigungsschritt nach dem ersten Reinigungsschritt erfolgen. Bevorzugt ist die Produktzufuhrleitung mit dem Tankelement verbunden und/oder an dem Tankelement angeordnet und zumindest dazu vorgesehen, zumindest das Produkt in das Tankelement zur Weiterverarbeitung, und zwar zum Abfüllen mittels der Dosiereinheit zu führen und/oder zu leiten. Zumindest zur Reinigung der Produktzufuhrleitung wird in dem zweiten Reinigungsschritt das Reinigungsmedium durch die Produktzufuhrleitung geleitet und/oder geführt. Die Fluideingangsleitung kann mit der Produktzufuhrleitung verbunden und/oder an der Produktzufuhrleitung angeordnet werden. In dem zweiten Reinigungsschritt kann das Reinigungsmedium von der CIP-Anlage durch die Fluideingangsleitung zu zumindest der Produktzufuhrleitung geleitet und/oder geführt werden.

Vorteilhaft unterscheidet sich der zweite Reinigungsschritt zu dem ersten Reinigungsschritt lediglich durch die Art und Weise, wie das Reinigungsmedium zum Tankelement geleitet und wie das Tankelement gereinigt wird. Insbesondere unterscheiden sich der erste Reinigungsschritt und der zweite Reinigungsschritt durch eine Anordnung und/oder Verbindung der Fluideingangsleitung mit der Produkttankeinheit. Während in dem ersten Reinigungsschritt das Reinigungsmedium durch die Fluideingangsleitung zu dem Reinigungsmittelzulauf der Produkttankeinheit und durch diese geleitet werden kann, kann in dem zweiten Reinigungsschritt das Reinigungsmedium durch die Fluideingangsleitung zu der Produktzufuhrleitung der Produkttankeinheit und durch diese geleitet werden.

Bevorzugt wird in zumindest dem zweiten Reinigungsschritt zumindest eine Verschmutzungskenngröße mittels des ersten Sensorelements sensiert und/oder erfasst. Die Steuereinheit kann in dem zweiten Reinigungsschritt den Grad der Verschmutzung und/oder Verunreinigung des Reinigungsmediums basierend auf zumindest der Verschmutzungskenngröße ermitteln und/oder analysieren. Vorteilhaft wird der zweite Reinigungsschritt, und zwar zumindest eine zeitliche Dauer des zweiten Reinigungsschritts mittels der Steuereinheit basierend auf dem Analyseergebnis gesteuert und/oder geregelt.

Außerdem wird vorgeschlagen, dass die Dosiervorrichtung eine Steuereinheit aufweist, welche dazu vorgesehen ist, die Verschmutzungskenngröße mit einer Reinigungsmittelausgangskenngröße sensiert durch ein zweites Sensorelement der Sensoreinheit an einem Zulauf der Abfülleinheit zu vergleichen. Hierdurch kann eine Reinigungseffizienz hinsichtlich einer Steuerung und/oder Regelung eines Reinigungsprozesses optimiert werden. Der Reinigungsprozess, und zwar zumindest eine zeitliche Dauer und/oder eine Verwendung von Reinigungsmedien kann/können individuell und flexibel angepasst und Ressourcen, und zwar zumindest das Reinigungsmedium und/oder Energie effizient genutzt werden. Zudem kann eine Ermittlung und/oder Analyse einer Verunreinigung und/oder Verschmutzung des Reinigungsmediums vereinfacht und/oder effizienter gestaltet werden.

Insbesondere handelt es sich bei der Steuereinheit um die bereits genannte Steuereinheit. Besonders bevorzugt ist das zweite Sensorelement an der Fluideingangsleitung angeordnet, insbesondere dann, wenn die Fluideingangsleitung Teil der Dosiervorrichtung ist. Das zweite Sensorelement kann die Reinigungsmittelausgangskenngröße des Reinigungsmediums sensieren, wenn das Reinigungsmittel von der CIP-Anlage zu der Abfülleinheit durch die Fluideingangsleitung geleitet wird. Insbesondere ist das Reinigungsmedium in der Fluideingangsleitung unverschmutzt und/oder frei von Verunreinigungen, und zwar bevorzugt frei von Produktrückständen. Vorteilhaft dient die Reinigungsmittelausgangskenngröße als Referenzwert bei Ermittlung und/oder Analyse der Verschmutzung und/oder Verunreinigung des Reinigungsmediums. Insbesondere ist die Steuereinheit dazu vorgesehen, eine Referenzwert-Analyse durchführen. Alternativ oder zusätzlich wäre auch denkbar, dass die Steuereinheit die ermittelte und/oder sensierte Verschmutzungskenngröße, beispielsweise des ersten Sensorelements, als einen Absolutwert erfasst und auswertet. Die Steuereinheit kann eine Absolutwert-Analyse durchführen.

Besonders bevorzugt wird in zumindest dem ersten Reinigungsschritt und/oder zumindest dem zweiten Reinigungsschritt die Reinigungsmittelausgangskenngröße mittels des zweiten Sensorelements sensiert und/oder erfasst. Die Steuereinheit kann in dem ersten Reinigungsschritt und/oder zumindest dem zweiten Reinigungsschritt den Grad der Verschmutzung und/oder Verunreinigung des Reinigungsmediums basierend auf dem Vergleich der Verschmutzungskenngröße mit der Reinigungsmittelausgangskenngröße ermitteln und/oder analysieren. Vorteilhaft wird der erste Reinigungsschritt und/oder zumindest der zweite Reinigungsschritt, und zwar zumindest eine zeitliche Dauer des ersten Reinigungsschritts und/oder eine zeitliche Dauer zumindest des zweiten Reinigungsschritts mittels der Steuereinheit basierend auf dem Analyseergebnis gesteuert und/oder geregelt.

In einer weiteren Ausgestaltung der Erfindung wird vorgeschlagen, dass ein drittes Sensorelement der Sensoreinheit in einem rückseitigen Ausgang einer Dosierpumpe der Dosiereinheit angeordnet ist. Dadurch kann eine Reinigung einer Dosiereinheit weiter optimiert und damit wiederum eine Reinigungseffizienz gesteigert werden. Zudem kann ein Reinigungsprozess je nach Bedarf und/oder Notwendigkeit der Reinigung der Dosiereinheit beispielsweise verkürzt oder verlängert werden, sodass die Reinigungseffizienz hinsichtlich einer zeitlichen Dauer verbessert werden kann. Zudem können Ressourcen zur Reinigung der Dosiereinheit effizient eingesetzt werden.

Insbesondere handelt es sich bei der Dosierpumpe um die bereits genannte Dosierpumpe. Vorzugsweise unterscheidet sich der rückseitige Ausgang von dem bereits genannten Produktausgang. Zur Dosierung und/oder zur Förderung des Produkts zu dem Produktausgang kann die Dosierpumpe zumindest eine Kolbeneinheit mit zumindest einem Kolben aufweisen. Bevorzugt weist die Kolbeneinheit den rückseitigen Ausgang auf. Das Reinigungsmedium ist durch die Kolbeneinheit zur Reinigung der Kolben und zumindest eines Gehäuses der Kolbeneinheit, in welchem der Kolben angeordnet ist, führbar und/oder leitbar. Das dritte Sensorelement kann zumindest eine Verschmutzungskenngröße des Reinigungsmediums, und zwar an dem rückseitigen Ausgang nach Durchlauf des Reinigungsmediums durch die Kolbeneinheit sensieren und/oder erfassen.

Hinsichtlich des Verfahrens zur Reinigung der Dosiervorrichtung wird zudem vorgeschlagen, dass in einem dritten Reinigungsschritt das Reinigungsmedium durch die Produktzufuhrleitung der Produkttankeinheit und zu einem rückseitigen Ausgang einer Dosierpumpe der Dosiereinheit geleitet wird. Dadurch kann eine Reinigungseffizienz hinsichtlich einer Reinigung einer Produkttankeinheit, und zwar zumindest einer Produktzufuhrleitung sowie einer Dosiereinheit, und zwar zumindest eines rückseitigen Ausgangs einer Dosierpumpe der Dosiereinheit optimiert werden. Zudem kann eine zeitliche Dauer eines Reinigungsprozesses, bevorzugt zumindest eines dritten Reinigungsschritts des Reinigungsprozesses, individuell und/oder flexibel je nach Verunreinigung und/oder Verschmutzung des Reinigungsmediums angepasst werden. Damit können wiederum Ressourcen, wie beispielsweise Energie und/oder Reinigungsmedien effizienter eingesetzt werden.

Insbesondere handelt es sich bei dem Ausgang um den bereits genannten Ausgang. Hinsichtlich des zeitlichen Verlaufs des Verfahrens kann der dritte Reinigungsschritt nach dem zweiten Reinigungsschritt erfolgen. Vorteilhaft wird in dem dritten Reinigungsschritt das Reinigungsmedium durch die Kolbeneinheit zur Reinigung der Kolben und zumindest des Gehäuses geleitet und/oder geführt. Das Reinigungsmedium kann anschließend aus dem rückseitigen Ausgang herausgeführt werden, wobei der rückseitige Ausgang bevorzugt mit der Fluidausgangsleitung verbunden ist, durch welche das Reinigungsmedium anschließend zurück zur CIP-Anlage geführt und/oder geleitet werden kann. Vorteilhaft wird der Produktausgang der Dosiereinheit in dem dritten Reinigungsschritt blockiert und/oder verschlossen, sodass das Reinigungsmedium bei Reinigung der Dosiereinheit durch den rückseitigen Ausgang austreten muss.

Vorteilhaft wird in zumindest dem dritten Reinigungsschritt zumindest eine Verschmutzungskenngröße mittels des dritten Sensorelements sensiert und/oder erfasst. Ferner kann in dem dritten Reinigungsschritt die Reinigungsmittelausgangskenngröße mittels des zweiten Sensorelements sensiert und/oder erfasst werden. Die Steuereinheit kann in dem dritten Reinigungsschritt den Grad der Verschmutzung und/oder Verunreinigung des Reinigungsmediums basierend auf dem Vergleich der Verschmutzungskenngröße mit der Reinigungsmittelausgangskenngröße ermitteln und/oder analysieren. Vorzugsweise steuert und/oder regelt die Steuereinheit basierend auf dem Analyseergebnis den dritten Reinigungsschritt, und zwar zumindest eine zeitliche Dauer des dritten Reinigungsschritts.

Wenn die Abfülleinheit eine Fluidausgleichsleitung aufweist, an welcher ein viertes Sensorelement der Sensoreinheit angeordnet ist, kann eine Reinigungseffizienz hinsichtlich einer Reinigung einer Fluidausgleichsleitung gesteigert werden. Zudem können Ressourcen, wie beispielsweise Energie und/oder Reinigungsmedien, effizient eingesetzt und vorteilhaft eingespart werden, und zwar je nachdem wie stark die Verunreinigung und/oder Verschmutzung der Fluidausgleichsleitung ist.

Die Fluidausgleichsleitung kann an dem Tankelement angeordnet und/oder montiert sein. Bevorzugt dient die Fluidausgleichsleitung zum Fluidniveauausgleich, und zwar zum Produktniveauausgleich in dem Tankelement, wenn sich das Produkt bei Abfüllung in dem Tankelement befindet, um vorteilhaft ein gleichmäßiges Abfüllen des Produkts bereitzustellen und/oder zu ermöglichen. Bei Abfüllung des Produkts kann es vorkommen, dass das Produkt zumindest abschnittsweise und/oder zumindest teilweise in die Fluidausgleichsleitung gelangt und/oder eindringt. Das vierte Sensorelement kann zumindest eine Verschmutzungskenngröße des Reinigungsmediums, und zwar an der Fluidausgleichsleitung nach einem Durchlauf des Reinigungsmediums durch die Tankeinheit und zumindest teilweisen Durchlauf die Fluidausgleichsleitung sensieren und/oder erfassen.

Hinsichtlich des Verfahrens zur Reinigung der Dosiervorrichtung wird ferner vorgeschlagen, dass in einem vierten Reinigungsschritt das Reinigungsmedium durch die Produktzufuhrleitung der Produkttankeinheit und zu einer Fluidausgleichsleitung der Abfülleinheit, welche an der Produkttankeinheit angeordnet ist, geleitet wird. Dadurch kann eine Reinigungseffizienz hinsichtlich einer Reinigung einer Produkttankeinheit, und zwar zumindest einer Produktzufuhrleitung der Produkttankeinheit sowie einer Fluidausgleichsleitung einer Abfülleinheit einer Dosiervorrichtung optimiert werden. Zudem kann eine zeitliche Dauer eines Reinigungsprozesses, bevorzugt zumindest eines vierten Reinigungsschritts des Reinigungsprozesses, individuell und/oder flexibel je nach Verunreinigung und/oder Verschmutzung des Reinigungsmediums angepasst werden. Damit können wiederum Ressourcen, wie beispielsweise Energie und/oder Reinigungsmedien effizienter eingesetzt werden.

Das Reinigungsmedium kann in dem vierten Reinigungsschritt durch die Fluidausgleichsleitung geleitet werden, um die Fluidausgleichsleitung zu reinigen und bevorzugt Produktreste aus der Fluidausgleichsleitung zu entfernen. In dem vierten Reinigungsschritt kann das Reinigungsmedium analog zu den Reinigungsschritten zwei und drei durch die Fluideingangsleitung zu der Produktzufuhrleitung und durch diese zu dem Tankelement geleitet und/oder geführt werden. Alternativ könnte das Reinigungsmedium in zumindest dem vierten Reinigungsschritt auch durch einen anderen und/oder weiteren Zugang zu dem Tankelement geleitet und/oder geführt werden. Vorzugsweise wird das Tankelement vollständig mit dem Reinigungsmedium gefüllt, und zwar überflutet, sodass das Reinigungsmedium in die Fluidausgleichsleitung gelangt und/oder eindringt. Insbesondere erfolgt der vierte Reinigungsschritt hinsichtlich des zeitlichen Verlaufs des Verfahrens nach dem dritten Reinigungsschritt.

Vorteilhaft wird in zumindest dem vierten Reinigungsschritt zumindest eine Verschmutzungskenngröße mittels des vierten Sensorelements sensiert und/oder erfasst. Ferner kann in dem dritten Reinigungsschritt die Reinigungsmittelausgangskenngröße mittels des zweiten Sensorelements sensiert und/oder erfasst werden. Die Steuereinheit kann in dem vierten Reinigungsschritt den Grad der Verschmutzung und/oder Verunreinigung des Reinigungsmediums basierend auf dem Vergleich der Verschmutzungskenngröße mit der Reinigungsmittelausgangskenngröße ermitteln und/oder analysieren. Vorzugsweise steuert und/oder regelt die Steuereinheit basierend auf dem Analyseergebnis den vierten Reinigungsschritt, und zwar zumindest eine zeitliche Dauer des vierten Reinigungsschritts.

Handelt es sich bei dem Reinigungsprozess um das Zwischenspülen, werden vorteilhaft lediglich der erste Reinigungsschritt und der zweite Reinigungsschritt durchgeführt. Handelt es sich bei dem Reinigungsprozess um den Endreinigungsprozess, werden besonders bevorzugt die Reinigungsschritte eins bis vier durchgeführt.

Die Dosiervorrichtung und/oder das Abfüllsystem und/oder das Verfahren soll/sollen hierbei nicht auf die oben beschriebene Anwendung und Ausführungsform beschränkt sein. Insbesondere kann/können die Dosiervorrichtung und/oder das Abfüllsystem und/oder das Verfahren zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in diesem Dokument angegebenen Wertebereichen auch innerhalb der genannten Grenzen liegende Werte als offenbart und als beliebig einsetzbar gelten.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine Dosiervorrichtung eines Abfüllsystems,
- Fig. 2: eine gedrehte Ansicht der Dosiervorrichtung gemäß der Figur 1,
- Fig. 3: eine weitere Ansicht der Dosiervorrichtung gemäß den Figuren 1 und 2,
- Fig. 4: ein schematisches Ablaufdiagramm eines Verfahrens zur Reinigung der Dosiervorrichtung gemäß den Figuren 1 bis 3,
- Fig. 5: einen ersten Reinigungsschritt des Verfahrens gemäß Figur 4,
- Fig. 6: einen zweiten Reinigungsschritt des Verfahrens gemäß Figur 4,
- Fig. 7: einen dritten Reinigungsschritt des Verfahrens gemäß Figur 4 und
- Fig. 8: einen vierten Reinigungsschritt des Verfahrens gemäß Figur 4.

### Beschreibung des Ausführungsbeispiels

Nachfolgend ist von mehrfach vorhandenen Objekten in den Figuren jeweils lediglich eines mit einem Bezugszeichen versehen. Ferner handelt es sich bei den vorliegenden Figuren um schematische und nicht maßstabsgetreue Darstellungen.

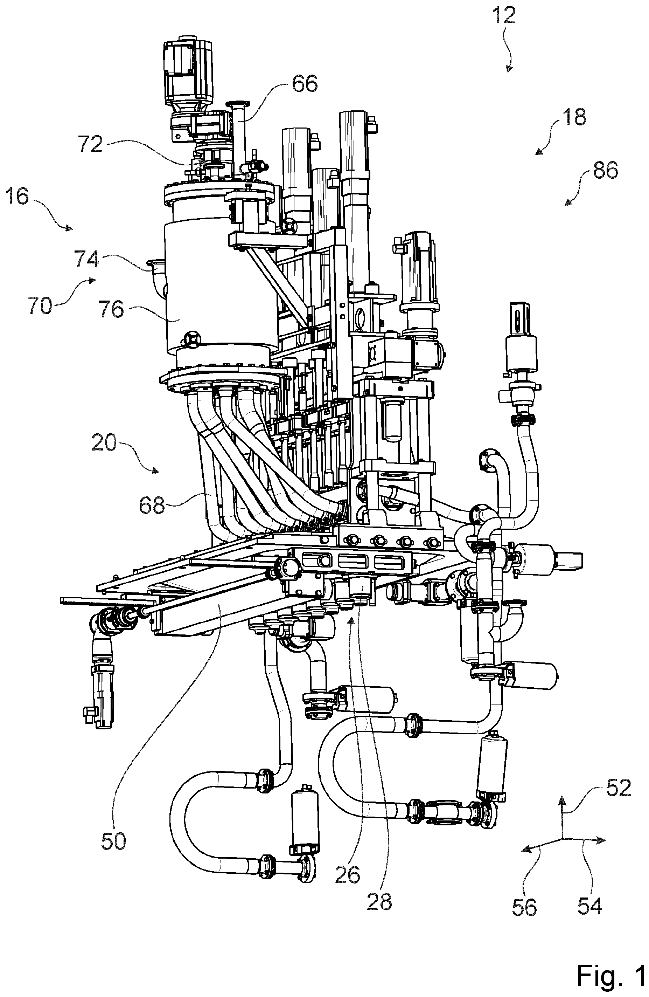

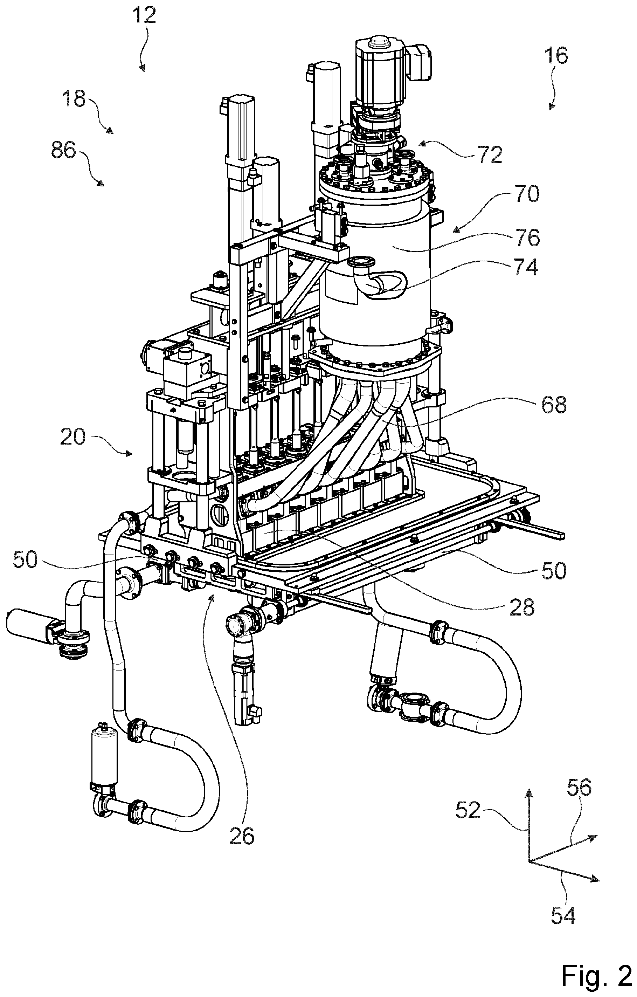

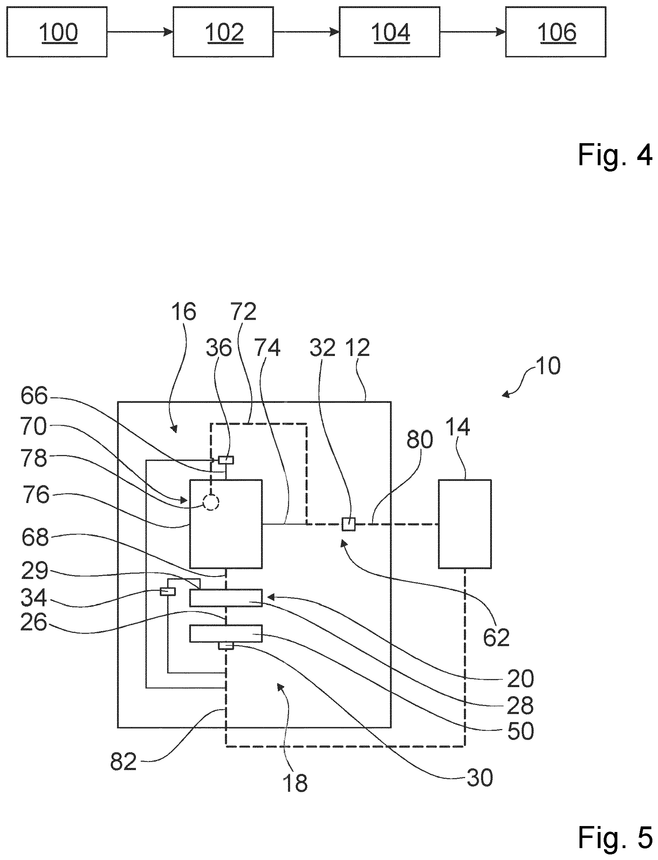

Die Figur 1 zeigt eine Dosiervorrichtung 12 eines Abfüllsystems 10. Vorliegend handelt es sich bei der Dosiervorrichtung 12 um eine Lebensmitteldosiervorrichtung. Die Dosiervorrichtung 12 ist zu Reinigungszwecken zu einer Verbindung mit einer CIP-Anlage 14 vorgesehen. Die CIP-Anlage 14 ist ebenfalls Teil des Abfüllsystems 10 (vgl. Figuren 5 bis 8). Bevor im Detail auf den Aufbau der Dosiervorrichtung 12 und die Reinigung der Dosiervorrichtung 12 eingegangen wird, sei angemerkt, dass die Figuren 1 bis 3 zur Verdeutlichung eines Aufbaus und einer Konstruktion der Dosiervorrichtung 12 dienen. Ferner zeigt die Figur 4 ein schematisches Ablaufdiagramm eines Verfahrens zur Reinigung der Dosiervorrichtung 12, wobei in den Figuren 5 bis 8 jeweils die einzelnen Reinigungsschritte des Verfahrens genauer dargestellt sind.

Die Dosiervorrichtung 12 weist eine Abfülleinheit 16 zur Förderung von zumindest flüssigen oder pastösen Produkten auf. Die Abfülleinheit 16 weist eine Produkttankeinheit 70 auf. Die Produkttankeinheit 70 umfasst zumindest ein und vorliegend genau ein Tankelement 76. Das Tankelement 76 ist als Tank ausgebildet.

Ferner weist die Abfülleinheit 16 eine Dosiereinheit 20 auf. Die Dosiereinheit 20 ist zur Dosierung und Abfüllung des Produkts in Produktbehälter (nicht dargestellt) vorgesehen. Vorliegend umfasst die Dosiereinheit 20 eine Vielzahl an Dosierpumpen 28, welche in dieser beispielhaften Ausführung in einer einzelnen Reihe nebeneinander angeordnet sind. Alternativ könnten die Dosierpumpen 28 beispielsweise auch matrix-förmig angeordnet sein. Die Dosierpumpen 28 sind einzeln über individuelle Zuführleitungen 68 der Dosiereinheit 20 mit der Produkttankeinheit 70, und zwar dem Tankelement 76 verbunden.

Die Dosiereinheit 20 weist einen Produktausgang 26 auf. Durch den Produktausgang 26 ist das Produkt in den Produktbehälter abfüllbar und/oder dosierbar. Vorliegend weist die Dosierpumpe 28 den Produktausgang 26 auf. Bei Abfüllung des Produkts ist das Produkt von der Produkttankeinheit 70 durch die Zuführleitungen 68 zur Dosiereinheit 20, und zwar zumindest zu den Dosierpumpen 28 förderbar und/oder leitbar, mittels welchen das Produkt durch den Produktausgang 26 in die Produktbehälter abfüllbar und/oder dosierbar ist.

Zur Einführung und/oder Einbringung des Produkts in das Tankelement 76 weist die Produkttankeinheit 70 eine Produktzufuhrleitung 74 auf. Die Produktzufuhrleitung 74 ist mit dem Tankelement 76 verbunden und/oder an dem Tankelement 76 angeordnet und dazu vorgesehen, zumindest das Produkt in das Tankelement 76 zur Weiterverarbeitung, und zwar zum Abfüllen mittels der Dosiereinheit 20 zu führen und/oder zu leiten.

In zumindest einer Produktionsphase füllt das Abfüllsystem 10, und zwar die Dosiervorrichtung 12 zumindest flüssige oder pastöse Produkte ab. Zur Reinigung zumindest der Abfülleinheit 16 ist die CIP-Anlage 14 über eine Schnittstelle der Dosiervorrichtung 12 mit der Abfülleinheit 16 verbindbar und/oder anschließbar. Die Schnittstelle der Dosiervorrichtung 12 zu der CIP-Anlage 14 besteht vorliegend aus zumindest einer Fluidleitung des Abfüllsystems 10, mittels welcher zumindest die Abfülleinheit 16 mit der CIP-Anlage 14 verbindbar und/oder an die CIP-Anlage 14 anschließbar ist. Das Abfüllsystem 10 weist zumindest eine Fluideingangsleitung 80, und zwar eine Reinigungsmitteleingangsleitung und zumindest eine Fluidausgangsleitung 82, und zwar eine Reinigungsmittelausgangsleitung auf, um die Abfülleinheit 16 mit der CIP-Anlage 14 zu verbinden. Unabhängig davon durch welche Elemente und/oder Einheiten und/oder Bereiche und/oder Bauteile der Abfülleinheit 16 das Reinigungsmedium geleitet oder geführt wird, ist das Reinigungsmedium stets durch die allgemeine Fluideingangsleitung 80 zur Abfülleinheit 16 leitbar und/oder führbar sowie durch die allgemeine Fluidausgangsleitung 82 von der Abfülleinheit 16 wieder ableitbar und/oder abführbar. Denkbar wäre auch, dass das Abfüllsystem 10 eine Vielzahl von Fluideingangs- und/oder Fluidausgangsleitungen 80, 82, und zwar zumindest zwei Fluideingangsleitungen 80 und/oder zumindest zwei Fluidausgangsleitungen 82 aufweist (nicht dargestellt). Ferner sind in dieser beispielhaften Ausgestaltung die Fluideingangsleitung 80 und die Fluidausgangsleitung 82 zumindest teilweise Teil der Dosiervorrichtung 12 und können zur Reinigung mit der Abfülleinheit 16 und der CIP-Anlage 14 verbunden werden.

In zumindest einem Reinigungsprozess reinigt die CIP-Anlage 14 die Abfülleinheit 16 mittels eines Reinigungsmediums. In dem Reinigungsprozess ist das Reinigungsmedium von der CIP-Anlage 14 durch die Fluideingangsleitung 80 zu der Abfülleinheit 16 führbar und/oder leitbar. Ferner ist in dem Reinigungsprozess das Reinigungsmedium von der Abfülleinheit 16 durch die Fluidausgangsleitung 82 wieder zurück zu der CIP-Anlage 14 führbar und/oder leitbar (vgl. Figuren 5 bis 8).

Der Reinigungsprozess kann sowohl ein sogenanntes Zwischenspülen, beispielsweise bei einem Produktwechsel innerhalb der Produktionsphase, als auch ein Endreinigungsprozess, beispielsweise am Ende der Produktionsphase, sein. Der als Zwischenspülen benannte Reinigungsprozess erfolgt zwischen dem jeweiligen Abfüllen der verschiedenen Produkte, um vorteilhaft Produktrückstände eines ersten Produkts vor einem Abfüllen eines zweiten Produkts zu entfernen. Bei dem Zwischenspülen kann sich das Reinigungsmedium zumindest zu einem Großteil und bevorzugt vollständig aus Wasser zusammensetzen. Bei dem Endreinigungsprozess werden jedoch Chemikalien und/oder zusätzliche Stoffe zur Reinigung verwendet, sodass das Reinigungsmedium bei Reinigung jene Chemikalien und/oder zusätzlichen Stoffe aufweist. Der Reinigungsprozess, und zwar der Endreinigungsprozess erfolgt am Ende der Produktionsphase, und zwar am Ende der Abfüllung von bevorzugt mehreren und unterschiedlichen Produkten, wie beispielsweise dem ersten Produkt und/oder zumindest dem zweiten Produkt.

Die Dosiervorrichtung 12 weist eine Steuereinheit 86 auf, welche dazu vorgesehen ist, zumindest den Reinigungsprozess zu steuern und/oder zu regeln. Vorliegend ist die Steuereinheit 86 zumindest zu einem Starten und/oder Beginnen und/oder Pausieren und/oder Aussetzen und/oder Beenden des Reinigungsprozesses vorgesehen. Ferner ist mittels der Steuereinheit 86 eine zeitliche Dauer und/oder ein zeitlicher Verlauf des Reinigungsprozesses steuerbar und/oder regelbar. Zusätzlich ist die Steuereinheit 86 zur Steuerung des Reinigungsprozesses dazu vorgesehen, die Produktionsphase, und zwar den Betrieb und das Abfüllen von Produkten mittels der Abfülleinheit 16 zu steuern und/oder zu regeln. Die Steuereinheit 86 kann das Abfüllen von Produkten beenden und den Reinigungsprozess starten. Beispielsweise kann die Steuereinheit 86 das Abfüllen des ersten Produkts beenden und das Zwischenspülen starten. Nach Beenden des Zwischenspülens kann die Steuereinheit 86 das Abfüllen des zweiten Produkts starten. Alternativ könnte die Steuereinheit 86 die gesamte Produktionsphase beenden und den Reinigungsprozess, und zwar den Endreinigungsprozess zumindest starten und steuern und/oder regeln.

Um eine Reinigung, und zwar den Reinigungsprozess zu optimieren und eine Reinigungseffizienz zu steigern, weist die Dosiervorrichtung 12 eine Sensoreinheit 18 zur Sensierung zumindest einer Verschmutzungskenngröße des Reinigungsmediums in dem Reinigungsprozess auf. Die Verschmutzungskenngröße charakterisiert und/oder definiert eine Verschmutzung und/oder Verunreinigung des Reinigungsmediums. Die Sensoreinheit 18 ist dazu vorgesehen, Rückstände von Produkten in dem Reinigungsmedium zu sensieren. Ferner ist die Sensoreinheit 18 zumindest dazu vorgesehen, einen Trübungsgrad des Reinigungsmediums zu sensieren. Zusätzlich kann die Sensoreinheit 18 zur Erfassung der Verschmutzung und/oder Verunreinigung des Reinigungsmediums weitere Kenngrößen, wie beispielsweise eine Temperatur, einen Volumenstrom, einen Leitwert oder dergleichen sensieren. Aufgrund der Erfassung und/oder Sensierung der Verschmutzungskenngröße mit der Sensoreinheit 18, ist mittels der Steuereinheit 86 ein Grad der Verschmutzung und/oder Verunreinigung des Reinigungsmediums ermittelbar. Die Steuereinheit 86 ist vorliegend dazu vorgesehen, den Reinigungsprozess basierend auf einem Analyseergebnis der Verschmutzungskenngröße zu steuern und/oder zu regeln. Basierend auf dem Analyseergebnis der Verschmutzungskenngröße kann die Steuereinheit 86 den Reinigungsprozess beenden und/oder verkürzen und/oder verlängern. Damit kann eine Steuerung des Reinigungsprozesses individuell und flexibel angepasst und vorteilhaft Ressourcen, und zwar das Reinigungsmedium effizient genutzt werden.

Die Sensoreinheit 18 könnte genau ein Sensorelement, beispielsweise ein optisches Sensorelement, aufweisen oder aus jenem bestehen. In dieser beispielhaften Ausgestaltung weist die Sensoreinheit 18 vier Sensorelemente 30, 32, 34, 36 auf, welche an unterschiedlichen Stellen und/oder Positionen innerhalb der Abfülleinheit 16 angeordnet sind. Die einzelnen Sensorelemente 30, 32, 34, 36 der Sensoreinheit 18 sensieren und/oder erfassen jeweils einzelne Kenngrößen des Reinigungsmediums, mittels welchen der Grad der Verschmutzung und/oder Verunreinigung des Reinigungsmediums ermittelbar ist. Dadurch kann eine präzise Aussage über die Verschmutzung und/oder Verunreinigung des Reinigungsmediums, und zwar bevorzugt an den jeweiligen unterschiedlichen Stellen innerhalb der Abfülleinheit 16, getroffen werden.

Ein erstes Sensorelement 30 der Sensoreinheit 18 ist fluidtechnisch hinter dem Produktausgang 26 der Dosiereinheit 20 angeordnet (vgl. Figuren 5 bis 8). Den Figuren 5 bis 8 ist zu entnehmen, dass das erste Sensorelement 30 zwar flugtechnisch hinter dem Produktausgang 26 der Dosiereinheit 20, aber fluidtechnisch vor der Fluidausgangsleitung 82 angeordnet ist. Alternativ und/oder zusätzlich wäre jedoch auch denkbar, dass die Fluidausgangsleitung 82 das erste Sensorelement 30 oder ein zusätzliches erstes Sensorelement 30' aufweist (nicht dargestellt).

Ferner weist die Dosiervorrichtung 12 zur Aufnahme des Reinigungsmediums nach Reinigung zumindest der Dosiereinheit 20 eine Aufnahmeeinheit 50 auf, an welcher das erste Sensorelement 30 angeordnet ist. Vorliegend handelt es sich bei der Aufnahmeeinheit 50 um eine sogenannte Spülplatte. Die Aufnahmeeinheit 50 nimmt das Reinigungsmedium nach Durchlauf durch den Produktausgang 26 und somit nach Reinigung der Dosiereinheit 20, und zwar zumindest der Dosierpumpe 28 auf.

Die Figuren 1 bis 3 verdeutlichen, dass die Aufnahmeeinheit 50 in Höhenrichtung 52 betrachtet unterhalb der Dosiereinheit 20 anordenbar ist, wobei die Figuren 1 bis 3 eine Positionierung der Aufnahmeeinheit 50 während der Produktionsphase darstellen. In dem Reinigungsprozess ist die Aufnahmeeinheit 50 in Höhenrichtung 52 betrachtet unterhalb den Dosierpumpen 28 anordenbar und/oder positionierbar, um das Reinigungsmedium nach Durchlauf durch den Produktausgang 26 aufzunehmen. Dafür ist zumindest die Aufnahmeeinheit 50 beweglich gelagert und (je nach Betrachtungsweise) in Breitenrichtung 54 und/oder in Tiefenrichtung 56 verschiebbar gelagert. Zur Anordnung der Aufnahmeeinheit 50 unterhalb der Dosierpumpen 28 sind die Dosierpumpen 28 in Höhenrichtung 52 verschiebbar gelagert. In dem Reinigungsprozess sind die Dosierpumpen 28 in Höhenrichtung 52 betrachtet verschiebbar, um die Aufnahmeeinheit 50 unterhalb der Dosierpumpen 28 anzuordnen und/oder zu platzieren.

Das erste Sensorelement 30 sensiert eine Verschmutzungskenngröße des Reinigungsmediums fluidtechnisch hinter dem Produktausgang 26. Die Sensoreinheit 18 weist ein zweites Sensorelement 32 auf, welches an einem Zulauf 62 der Abfülleinheit 16 angeordnet ist. Das zweite Sensorelement 32 sensiert und/oder ermittelt eine Reinigungsmittelausgangskenngröße. Das zweite Sensorelement 32 sensiert die Reinigungsmittelausgangskenngröße des Reinigungsmediums, wenn das Reinigungsmittel von der CIP-Anlage 14 zu der Abfülleinheit 16 durch die Fluideingangsleitung 80 geleitet wird. In der Fluideingangsleitung 80 ist das Reinigungsmedium unverschmutzt und/oder frei von Verunreinigungen, und zwar bevorzugt frei von Produktrückständen.

Die Steuereinheit 86 könnte die ermittelte und/oder sensierte Verschmutzungskenngröße, beispielsweise des ersten Sensorelements 30, als einen Absolutwert erfassen und auswerten. Dabei kann die Steuereinheit 86 eine Absolutwert-Analyse durchführen. Alternativ oder zusätzlich ist die Steuereinheit 86 auch dazu vorgesehen, die ermittelte und/oder sensierte Verschmutzungskenngröße, beispielsweise des ersten Sensorelements 30, in Relation zu zumindest einer weiteren ermittelten und/oder sensierten Kenngröße innerhalb der Dosiervorrichtung 12 zu setzen. Ferner ist die Steuereinheit 86 zu einer Relationswert-Analyse vorgesehen. Um den Reinigungsprozess jedoch weiter zu verbessern, ist die Steuereinheit 86 dazu vorgesehen, die Verschmutzungskenngröße mit der Reinigungsmittelausgangskenngröße zu vergleichen. Damit kann die Steuereinheit 86 den Reinigungsprozess exakter steuern und/oder regeln, um bevorzugt vollständig Produktreste aus der Abfülleinheit 16 zu entfernen und die Abfülleinheit 16 vollständig zu reinigen.

Ferner weist die Sensoreinheit 18 ein drittes Sensorelement 34 auf, welches an einem rückseitigen Ausgang 29 der Dosierpumpe 28 der Dosiereinheit 20 angeordnet ist. Das dritte Sensorelement 34 sensiert ebenfalls eine Verschmutzungskenngröße des Reinigungsmediums, und zwar an dem rückseitigen Ausgang 29.

Zum Ausgleichen eines Produktspiegels in dem Tankelement 76, weist die Abfülleinheit 16 eine Fluidausgleichsleitung 66 auf, an welcher ein viertes Sensorelement 36 der Sensoreinheit 18 angeordnet ist. Das vierte Sensorelement 36 sensiert ebenfalls eine Verschmutzungskenngröße des Reinigungsmediums, und zwar an der Fluidausgleichsleitung 66. Die Fluidausgleichsleitung 66 ist an dem Tankelement 76 angeordnet und/oder montiert. Die Fluidausgleichsleitung 66 dient vorliegend zum Fluidniveauausgleich, und zwar zum Produktniveauausgleich in dem Tankelement 76, wenn sich das Produkt bei Abfüllung in dem Tankelement 76 befindet, um bevorzugt ein gleichmäßiges Abfüllen des Produkts bereitzustellen. Bei Abfüllung des Produkts kann es vorkommen, dass das Produkt zumindest abschnittsweise und/oder zumindest teilweise in die Fluidausgleichsleitung 66 gelangt und/oder eindringt.

Vorliegend ist die Steuereinheit 86 dazu vorgesehen, jede Verschmutzungskenngröße des Reinigungsmediums, sensiert und/oder erfasst durch das erste Sensorelement 30, das dritte Sensorelement 34 und/oder das vierte Sensorelement 36, mit der Reinigungsmittelausgangskenngröße, sensiert und/oder ermittelt durch das zweite Sensorelement 32, zu vergleichen. Basierend auf dem Analyseergebnis steuert und/oder regelt die Steuereinheit 86 den Reinigungsprozess.

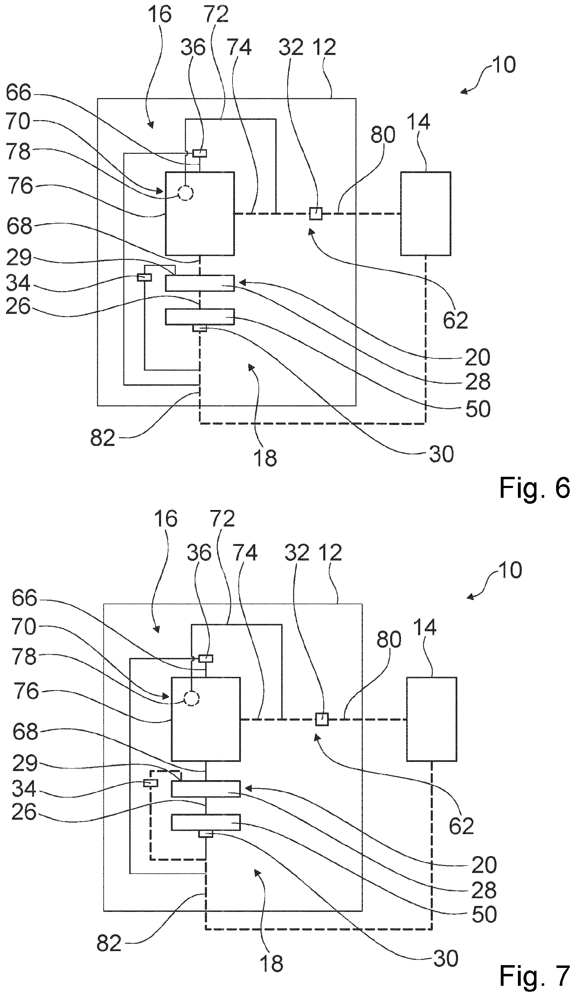

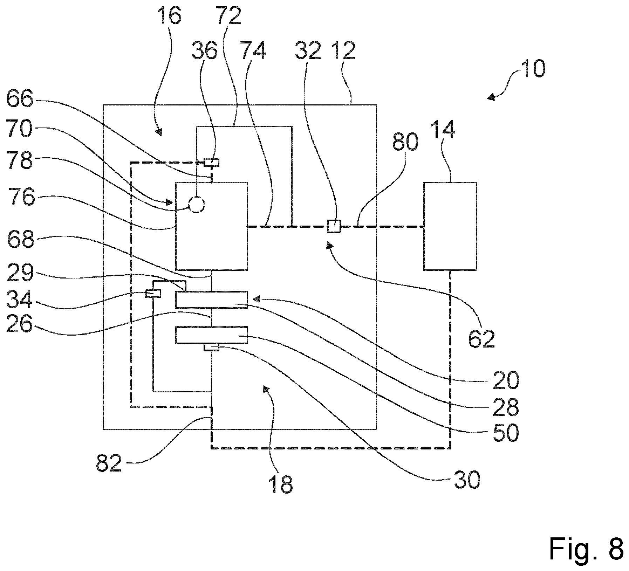

In dem Verfahren zur Reinigung der Dosiervorrichtung 12 wird in dem Reinigungsprozess zumindest die Verschmutzungskenngröße des Reinigungsmediums sensiert. Das Verfahren zur Reinigung der Dosiervorrichtung 12 ist beispielhaft in den Figuren 4 bis 8 verdeutlicht. Das Verfahren kann mehrere Verfahrensschritte und Verfahrensteilschritte umfassen. Vorliegend weist das Verfahren zumindest vier Reinigungsschritte 100, 102, 104, 106 auf. Die Figur 5 zeigt den ersten Reinigungsschritt 100, die Figur 6 den zweiten Reinigungsschritt 102, die Figur 7 den dritten Reinigungsschritt 104 und die Figur 8 den vierten Reinigungsschritt 106. Vorliegend erfolgt der erste Reinigungsschritt 100 hinsichtlich eines zeitlichen Verlaufs des Verfahrens vor dem zweiten Reinigungsschritt 102, der dritte Reinigungsschritt 104 nach dem zweiten Reinigungsschritt 102 und der vierte Reinigungsschritt 106 nach dem dritten Reinigungsschritt 104. Die einzelnen Reinigungsschritte werden im Folgenden genauer erläutert.

In dem ersten Reinigungsschritt 100 wird das Reinigungsmedium durch einen Reinigungsmittelzulauf 72 der Produkttankeinheit 70 zu dem Produktausgang 26 der Dosiereinheit 20 geleitet (vgl. Figur 5). Das Reinigungsmedium wird in dem ersten Reinigungsschritt 100 durch die Fluideingangsleitung 80 zu dem Reinigungsmittelzulauf 72 geleitet und/oder geführt. Vorliegend ist der Reinigungsmittelzulauf 72 mit dem Tankelement 76 verbunden und/oder an dem Tankelement 76 angeordnet. Der Reinigungsmittelzulauf 72 kann beispielsweise zumindest eine Leitung zur Leitung und/oder Führung des Reinigungsmediums von der Fluideingangsleitung 80 zu zumindest dem Tankelement 76 aufweisen. In dieser beispielhaften Ausgestaltung umfasst der Reinigungsmittelzulauf 72 zumindest eine bevorzugt rotierbare und/oder bewegbare oder stationäre Sprühkugel 78. Zumindest die Sprühkugel 78 ist innerhalb des Tankelements 76 angeordnet und in dem ersten Reinigungsschritt 100 innerhalb des Tankelements 76 bewegbar, und zwar zumindest rotierbar. Die Sprühkugel 78 kann sich in Höhenrichtung 52, in Breitenrichtung 54 und/oder in Tiefenrichtung 56 innerhalb des Tankelements 76 bewegen, um das Tankelement 76 zu reinigen.

Ferner wird in dem ersten Reinigungsschritt 100 das Reinigungsmedium von dem Reinigungsmittelzulauf 72 durch das Tankelement 76 zur Reinigung des Tankelements 76, und anschließend zur Reinigung der Dosiereinheit 20 zu der Dosiereinheit 20, und zwar durch die Zuführleitungen 68 von dem Tankelement 76 zu den Dosierpumpen 28, ferner zu dem Produktausgang 26 der Dosiereinheit 20, geleitet und/oder geführt. Das Reinigungsmedium, welches am Produktausgang 26 austritt, wird in dem ersten Reinigungsschritt 100 anschließend von der Aufnahmeeinheit 50 aufgenommen und durch die Fluidausgangsleitung 82 zurück zur CIP-Anlage 14 geführt. In zumindest dem ersten Reinigungsschritt 100 wird die Verschmutzungskenngröße mittels des ersten Sensorelements 30 sensiert und/oder erfasst. Ferner wird in dem ersten Reinigungsschritt 100 die Reinigungsmittelausgangskenngröße mittels des zweiten Sensorelements 32 sensiert und/oder erfasst. Die Steuereinheit 86 ermittelt vorliegend in dem ersten Reinigungsschritt 100 den Grad der Verschmutzung und/oder Verunreinigung des Reinigungsmediums basierend auf dem Vergleich der Verschmutzungskenngröße mit der Reinigungsmittelausgangskenngröße. Basierend auf dem Analyseergebnis steuert und/oder regelt die Steuereinheit 86 den ersten Reinigungsschritt 100, und zwar zumindest eine zeitliche Dauer des ersten Reinigungsschritts 100.

Zur Reinigung zumindest der Produktzufuhrleitung 74 wird in dem zweiten Reinigungsschritt 102 das Reinigungsmedium durch die Produktzufuhrleitung 74 der Produkttankeinheit 70 zu dem Produktausgang 26 der Dosiereinheit 20 geleitet. Figur 6 verdeutlicht, dass in dem zweiten Reinigungsschritt 102, das Reinigungsmedium durch die Fluideingangsleitung 80 zu der Produktzufuhrleitung 74 und durch diese zu dem Tankelement 76 geleitet und/oder geführt wird. In dem zweiten Reinigungsschritt 102 werden das Tankelement 76 und die Dosiereinheit 20 ebenfalls zusätzlich gereinigt. Der zweite Reinigungsschritt 102 unterscheidet sich zu dem ersten Reinigungsschritt 100 vorliegend lediglich durch die Art und Weise, wie das Reinigungsmedium zum Tankelement 76 geleitet und wie das Tankelement 76 gereinigt wird. Dabei unterscheiden sich der ersten Reinigungsschritt 100 und der zweite Reinigungsschritt 102 durch eine Anordnung und/oder Verbindung der Fluideingangsleitung 80 mit der Produkttankeinheit 70. Während in dem ersten Reinigungsschritt 100 das Reinigungsmedium durch die Fluideingangsleitung 80 zu dem Reinigungsmittelzulauf 72 der Produkttankeinheit 70 und durch diese zu dem Tankelement 76 geleitet wird, wird in dem zweiten Reinigungsschritt 102 das Reinigungsmedium durch die Fluideingangsleitung 80 zu der Produktzufuhrleitung 74 der Produkttankeinheit 70 und durch diese zu dem Tankelement 76 geleitet.

In zumindest dem zweiten Reinigungsschritt 102 wird die Verschmutzungskenngröße mittels des ersten Sensorelements 30 sensiert und/oder erfasst. Ferner wird in zumindest dem zweiten Reinigungsschritt 102 die Reinigungsmittelausgangskenngröße mittels des zweiten Sensorelements 32 sensiert und/oder erfasst. Die Steuereinheit 86 ermittelt vorliegend in dem zweiten Reinigungsschritt 102 den Grad der Verschmutzung und/oder Verunreinigung des Reinigungsmediums basierend auf dem Vergleich der Verschmutzungskenngröße mit der Reinigungsmittelausgangskenngröße. Basierend auf dem Analyseergebnis steuert und/oder regelt die Steuereinheit 86 den zweiten Reinigungsschritt 102, und zwar zumindest eine zeitliche Dauer des zweiten Reinigungsschritts 102.

Handelt es sich bei dem Reinigungsprozess um das Zwischenspülen, werden lediglich der erste Reinigungsschritt 100 und der zweite Reinigungsschritt 102 durchgeführt. Handelt es sich bei dem Reinigungsprozess um den Endreinigungsprozess, werden zusätzlich zu den Reinigungsschritten eins 100 und zwei 102 noch die Reinigungsschritte drei 104 und vier 106 durchgeführt.

In dem dritten Reinigungsschritt 104 gemäß Figur 7 wird das Reinigungsmedium durch die Produktzufuhrleitung 74 der Produkttankeinheit 70 und zu einem rückseitigen Ausgang 29 der Dosierpumpen 28 der Dosiereinheit 20 geleitet. Gemäß Figur 7 ist ersichtlich, dass die Zufuhr des Reinigungsmediums von der CIP-Anlage 14 zu der Produkttankeinheit 70 in dem dritten Reinigungsschritt 104 analog zu dem zweiten Reinigungsschritt 102 erfolgt. Die Reinigungsschritte zwei 102 und drei 104 unterscheiden sich in dieser beispielhaften Ausführung hinsichtlich eines Ablaufs, und zwar einer Führung und/oder Leitung des Reinigungsmediums nach dem Tankelement 76. Zwar wird in dem dritten Reinigungsschritt 104 das Reinigungsmedium ebenfalls durch die Zuführleitungen 68 von dem Tankelement 76 zu den Dosierpumpen 28 geführt, jedoch unterscheidet sich der rückseitige Ausgang 29 der Dosierpumpen 28 von dem bereits genannten Produktausgang 26 der Dosierpumpen 28.

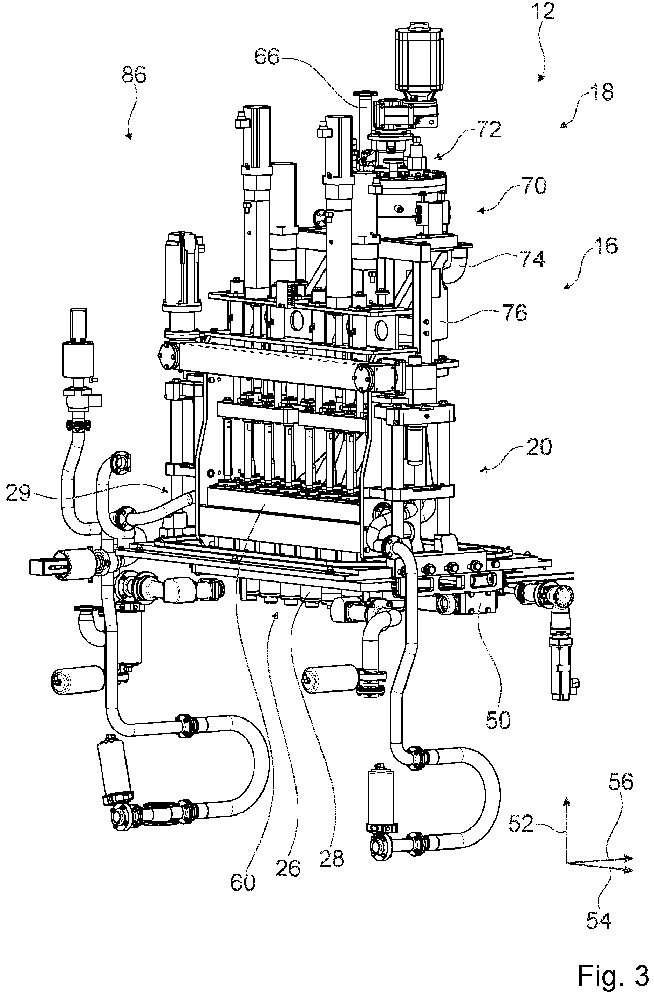

Zur Dosierung und/oder zur Förderung des Produkts zu dem Produktausgang 26 weist die Dosierpumpe 28 zumindest eine Kolbeneinheit 60 mit zumindest einem Kolben auf. Vorliegend weist die Kolbeneinheit 60 den rückseitigen Ausgang 29 auf (vgl. Figur 3). In dem dritten Reinigungsschritt 104 wird das Reinigungsmedium durch die Kolbeneinheit 60 zur Reinigung der Kolben und zumindest eines Gehäuses der Kolbeneinheit 60, in welchem der Kolben angeordnet ist, geleitet und/oder geführt. Das Reinigungsmedium wird anschließend aus dem rückseitigen Ausgang 29 herausgeführt. Der rückseitige Ausgang 29 ist mit der Fluidausgangsleitung 82 verbunden, durch welche das Reinigungsmedium anschließend zurück zur CIP-Anlage 14 geführt und/oder geleitet wird. In dieser beispielhaften Ausgestaltung wird der Produktausgang 26 in dem dritten Reinigungsschritt 104 blockiert und/oder verschlossen, sodass das Reinigungsmedium bei Reinigung der Dosiereinheit 20 durch den rückseitigen Ausgang 29 austreten muss.

In zumindest dem dritten Reinigungsschritt 104 wird die Verschmutzungskenngröße mittels des dritten Sensorelements 34 sensiert und/oder erfasst. Ferner wird in dem dritten Reinigungsschritt 104 die Reinigungsmittelausgangskenngröße mittels des zweiten Sensorelements 32 sensiert und/oder erfasst. Die Steuereinheit 86 ermittelt vorliegend in dem dritten Reinigungsschritt 104 den Grad der Verschmutzung und/oder Verunreinigung des Reinigungsmediums basierend auf dem Vergleich der Verschmutzungskenngröße mit der Reinigungsmittelausgangskenngröße. Basierend auf dem Analyseergebnis steuert und/oder regelt die Steuereinheit 86 den dritten Reinigungsschritt 104, und zwar zumindest eine zeitliche Dauer des dritten Reinigungsschritts 104.

In dem vierten Reinigungsschritt 106 wird das Reinigungsmedium ebenfalls durch die Produktzufuhrleitung 74 der Produkttankeinheit 70 und dann anschließend zu der Fluidausgleichsleitung 66 der Abfülleinheit 16 geleitet, welche an der Produkttankeinheit 70 angeordnet ist. Figur 8 zeigt, dass das Reinigungsmedium in dem vierten Reinigungsschritt 106 durch die Fluidausgleichsleitung 66 geleitet wird, um die Fluidausgleichsleitung 66 zu reinigen und bevorzugt Produktreste aus der Fluidausgleichsleitung 66 zu entfernen. In dem vierten Reinigungsschritt 106 wird das Reinigungsmedium analog zu den Reinigungsschritten zwei 102 und drei 104 durch die Fluideingangsleitung 80 zu der Produktzufuhrleitung 76 und durch diese zu dem Tankelement 76 geleitet und/oder geführt. Das Tankelement 76 wird vollständig mit dem Reinigungsmedium gefüllt, und zwar überflutet, sodass das Reinigungsmedium in die Fluidausgleichsleitung 66 gelangt.

Ferner wird in zumindest dem vierten Reinigungsschritt 106 die Verschmutzungskenngröße mittels des vierten Sensorelements 36 sensiert und/oder erfasst. Zusätzlich wird in dem vierten Reinigungsschritt 106 die Reinigungsmittelausgangskenngröße mittels des zweiten Sensorelements 32 sensiert und/oder erfasst. Die Steuereinheit 86 ermittelt vorliegend in dem vierten Reinigungsschritt 106 den Grad der Verschmutzung und/oder Verunreinigung des Reinigungsmediums basierend auf dem Vergleich der Verschmutzungskenngröße mit der Reinigungsmittelausgangskenngröße. Basierend auf dem Analyseergebnis steuert und/oder regelt die Steuereinheit 86 den vierten Reinigungsschritt 106, und zwar zumindest eine zeitliche Dauer des vierten Reinigungsschritts 106.

## Patentansprüche

1. Dosiervorrichtung (12), insbesondere Lebensmitteldosiervorrichtung, mit zumindest einer Abfülleinheit (16) zur Förderung von zumindest flüssigen oder pastösen Produkten, mit einer Schnittstelle zu einer CIP-Anlage (14) zur Reinigung der Abfülleinheit (16) mittels eines Reinigungsmediums in zumindest einem Reinigungsprozess, mit einer Sensoreinheit (18) zur Sensierung zumindest einer Verschmutzungskenngröße des Reinigungsmediums in dem Reinigungsprozess, und mit einer Aufnahmeeinheit (50) zur Aufnahme des Reinigungsmediums, an welcher ein erstes Sensorelement (30) der Sensoreinheit (18) angeordnet ist, **dadurch gekennzeichnet, dass** die Abfülleinheit (16) eine Dosiereinheit (20) aufweist und das erste Sensorelement (30) der Sensoreinheit (18) fluidtechnisch hinter einem Produktausgang (26) der Dosiereinheit (20) angeordnet ist, wobei die Aufnahmeeinheit (50) zur Aufnahme des Reinigungsmediums nach einer Reinigung zumindest der Dosiereinheit (20) vorgesehen ist, wobei die Dosiereinheit (20) zumindest eine Dosierpumpe (28) aufweist, welche zur Dosierung der Produkte vorgesehen ist, wobei die Dosierpumpe (28) den Produktausgang (26) aufweist.

2. Dosiervorrichtung (12) nach Anspruch 1, **dadurch gekennzeichnet, dass** die Sensoreinheit (18) dazu vorgesehen ist, Rückstände von Produkten in dem Reinigungsmedium zu sensieren.

3. Dosiervorrichtung (12) nach Anspruch 1 oder 2, **dadurch gekennzeichnet, dass** die Sensoreinheit (18) dazu vorgesehen ist, einen Trübungsgrad des Reinigungsmediums zu sensieren.

4. Dosiervorrichtung (12) nach einem der vorhergehenden Ansprüche,
**dadurch gekennzeichnet, dass** in dem Reinigungsprozess die Aufnahmeeinheit (50) in Höhenrichtung (52) betrachtet unterhalb der Dosiereinheit (20) anordenbar ist.

5. Dosiervorrichtung (12) nach einem der vorhergehenden Ansprüche, **gekennzeichnet durch** eine Steuereinheit (86), welche dazu vorgesehen ist, die Verschmutzungskenngröße mit einer Reinigungsmittelausgangskenngröße sensiert durch ein zweites Sensorelement (32) der Sensoreinheit (18) an einem Zulauf (62) der Abfülleinheit (16) zu vergleichen.

6. Dosiervorrichtung (12) nach einem der vorhergehenden Ansprüche,
**dadurch gekennzeichnet, dass** ein drittes Sensorelement (34) der Sensoreinheit (18) an einem rückseitigen Ausgang (29) einer Dosierpumpe (28) der Dosiereinheit (20) angeordnet ist.

7. Dosiervorrichtung (12) nach einem der vorhergehenden Ansprüche,
**dadurch gekennzeichnet, dass** die Abfülleinheit (16) eine Fluidausgleichsleitung (66) aufweist, an welcher ein viertes Sensorelement (36) der Sensoreinheit (18) angeordnet ist.

8. Abfüllsystem (10) mit zumindest einer Dosiervorrichtung (12) nach einem der vorhergehenden Ansprüche und mit einer CIP-Anlage (14) zur Reinigung der Abfülleinheit mittels eines Reinigungsmediums in zumindest einem Reinigungsprozess.

9. Verfahren zur Reinigung einer Dosiervorrichtung (12), insbesondere nach einem der Ansprüche 1 bis 7, mit einer Abfülleinheit (16) zur Förderung von zumindest flüssigen oder pastösen Produkten und mit einer Schnittstelle zu einer CIP-Anlage (14) zur Reinigung der Abfülleinheit (16) mittels eines Reinigungsmediums in zumindest einem Reinigungsprozess, wobei in dem Reinigungsprozess zumindest eine Verschmutzungskenngröße des Reinigungsmediums sensiert wird, wobei die Verschmutzungskenngröße des Reinigungsmediums durch ein erstes an einer Aufnahmeeinheit (50) der Dosiervorrichtung (12) angeordnetes Sensorelement (30) einer Sensoreinheit (18) der Dosiervorrichtung (12) sensiert und/oder erfasst wird, **dadurch gekennzeichnet, dass** die Aufnahmeeinheit (50) das Reinigungsmedium nach einer Reinigung zumindest einer zumindest eine Dosierpumpe (28) zu einer Dosierung von Produkten umfassenden Dosiereinheit (20) der Dosiervorrichtung (12) aufnimmt, wobei in einem ersten Reinigungsschritt (100) das Reinigungsmedium durch einen Reinigungsmittelzulauf (72) einer Produkttankeinheit (70) der Abfülleinheit (16) zu einem Produktausgang (26) der Dosiereinheit (20) geleitet wird, wobei die Dosierpumpe (28) den Produktausgang (26) aufweist.

10. Verfahren nach Anspruch 9, **dadurch gekennzeichnet, dass** in einem zweiten Reinigungsschritt (102) das Reinigungsmedium durch eine Produktzufuhrleitung (74) der Produkttankeinheit (70) zu dem Produktausgang (26) der Dosiereinheit (20) geleitet wird.

11. Verfahren nach einem der Ansprüche 9 oder 10, **dadurch gekennzeichnet, dass** in einem dritten Reinigungsschritt (104) das Reinigungsmedium durch die Produktzufuhrleitung (74) der Produkttankeinheit (70) und zu einem rückseitigen Ausgang (29) der Dosierpumpe (28) der Dosiereinheit (20) geleitet wird.

12. Verfahren nach einem der Ansprüche 9 bis11, **dadurch gekennzeichnet, dass** in einem vierten Reinigungsschritt (106) das Reinigungsmedium durch die Produktzufuhrleitung (74) der Produkttankeinheit (70) und zu einer Fluidausgleichsleitung (66) der Abfülleinheit (16), welche an der Produkttankeinheit (70) angeordnet ist, geleitet wird.

## Claims

1. A dosing device (12), in particular food dosing device, having at least one filling unit (16) for conveying at least liquid or pasty products, having an interface to a CIP system (14) for cleaning the filling unit (16) by means of a cleaning medium in at least one cleaning process, having a sensor unit (18) for sensing at least one contamination parameter of the cleaning medium in the cleaning process, and having a receiving unit (50) for receiving the cleaning medium, on which a first sensor element (30) of the sensor unit (18) is arranged, **characterized in that** the filling unit (16) has a dosing unit (20) and the first sensor element (30) of the sensor unit (18) is arranged fluidically downstream of a product outlet (26) of the dosing unit (20), wherein the receiving unit (50) is configured for receiving the cleaning medium after cleaning at least of the dosing unit (20), wherein the dosing unit (20) has at least one dosing pump (28) which is configured for dosing the products, wherein the dosing pump (28) has the product outlet (26).

2. The dosing device (12) according to claim 1, **characterized in that** the sensor unit (18) is configured for sensing residues of products in the cleaning medium.

3. The dosing device (12) according to claim 1 or 2, **characterized in that** the sensor unit (18) is configured for sensing a degree of turbidity of the cleaning medium.

4. The dosing device (12) according to any one of the preceding claims, **characterized in that,** in the cleaning process, the receiving unit (50) can be arranged below the dosing unit (20) as viewed in the height direction (52).

5. The dosing device (12) according to any one of the preceding claims, **characterized by** a control unit (86) which is configured for comparing the contamination parameter with a cleaning agent output parameter sensed by a second sensor element (32) of the sensor unit (18) at an inlet (62) of the filling unit (16).

6. The dosing device (12) according to any one of the preceding claims, **characterized in that** a third sensor element (34) of the sensor unit (18) is arranged at a rear outlet (29) of a dosing pump (28) of the dosing unit (20).

7. The dosing device (12) according to any one of the preceding claims, **characterized in that** the filling unit (16) has a fluid equalization line (66) on which a fourth sensor element (36) of the sensor unit (18) is arranged.

8. A filling system (10) having at least one dosing device (12) according to any one of the preceding claims and having a CIP system (14) for cleaning the filling unit by means of a cleaning medium in at least one cleaning process.

9. A method for cleaning a dosing device (12), in particular according to any one of claims 1 to 7, having a filling unit (16) for conveying at least liquid or pasty products and having an interface to a CIP system (14) for cleaning the filling unit (16) by means of a cleaning medium in at least one cleaning process, wherein at least one contamination parameter of the cleaning medium is sensed in the cleaning process, wherein the contamination parameter of the cleaning medium is sensed and/or detected by a first sensor element (30) of a sensor unit (18) of the dosing device (12) arranged on a receiving unit (50) of the dosing device (12), **characterized in that** the receiving unit (50) receives the cleaning medium after cleaning at least of a dosing unit (20) of the dosing device (12) comprising at least one dosing pump (28) for dosing products, wherein, in a first cleaning step (100), the cleaning medium is conducted through a cleaning agent inlet (72) of a product tank unit (70) of the filling unit (16) to a product outlet (26) of the dosing unit (20), wherein the dosing pump (28) has the product outlet (26).

10. The method according to claim 9, **characterized in that,** in a second cleaning step (102), the cleaning medium is conducted through a product supply line (74) of the product tank unit (70) to the product outlet (26) of the dosing unit (20).

11. The method according to any one of claims 9 or 10, **characterized in that,** in a third cleaning step (104), the cleaning medium is conducted through the product supply line (74) of the product tank unit (70) and to a rear outlet (29) of the dosing pump (28) of the dosing unit (20).

12. The method according to any one of claims 9 to 11, **characterized in that,** in a fourth cleaning step (106), the cleaning medium is conducted through the product supply line (74) of the product tank unit (70) and to a fluid equalization line (66) of the filling unit (16) which is arranged on the product tank unit (70).

## Revendications

1. Dispositif de dosage (12), en particulier dispositif de dosage d'aliments, comprenant au moins une unité de remplissage (16) pour le transport de produits au moins liquides ou pâteux, comprenant une interface avec une installation CIP (14) pour le nettoyage de l'unité de remplissage (16) au moyen d'un milieu de nettoyage dans au moins un processus de nettoyage, comprenant une unité de capteur (18) pour la détection d'au moins une grandeur caractéristique d'encrassement du milieu de nettoyage dans le processus de nettoyage, et comprenant une unité de réception (50) pour la réception du milieu de nettoyage, sur laquelle est disposé un premier élément de capteur (30) de l'unité de capteur (18), **caractérisé en ce que** l'unité de remplissage (16) présente une unité de dosage (20) et le premier élément de capteur (30) de l'unité de capteur (18) est disposé fluidiquement derrière une sortie de produit (26) de l'unité de dosage (20), l'unité de réception (50) étant prévue pour la réception du milieu de nettoyage après un nettoyage au moins de l'unité de dosage (20), l'unité de dosage (20) présentant au moins une pompe de dosage (28) qui est prévue pour le dosage des produits, la pompe de dosage (28) présentant la sortie de produit (26).

2. Dispositif de dosage (12) selon la revendication 1, **caractérisé en ce que** l'unité de capteur (18) est prévue pour détecter des résidus de produits dans le milieu de nettoyage.

3. Dispositif de dosage (12) selon la revendication 1 ou 2, **caractérisé en ce que** l'unité de capteur (18) est prévue pour détecter un degré de turbidité du milieu de nettoyage.

4. Dispositif de dosage (12) selon l'une quelconque des revendications précédentes, **caractérisé en ce que** dans le processus de nettoyage, l'unité de réception (50) peut être disposée, vue dans le sens de la hauteur (52), en dessous de l'unité de dosage (20).

5. Dispositif de dosage (12) selon l'une quelconque des revendications précédentes, **caractérisé par** une unité de commande (86) qui est prévue pour comparer la grandeur caractéristique d'encrassement à une grandeur caractéristique de sortie d'agent de nettoyage détectée par un deuxième élément de capteur (32) de l'unité de capteur (18) au niveau d'une entrée (62) de l'unité de remplissage (16).

6. Dispositif de dosage (12) selon l'une quelconque des revendications précédentes, **caractérisé en ce qu'**un troisième élément de capteur (34) de l'unité de capteur (18) est disposé au niveau d'une sortie arrière (29) d'une pompe de dosage (28) de l'unité de dosage (20).

7. Dispositif de dosage (12) selon l'une quelconque des revendications précédentes, **caractérisé en ce que** l'unité de remplissage (16) présente une conduite d'équilibrage de fluide (66), au niveau de laquelle est disposé un quatrième élément de capteur (36) de l'unité de capteur (18).

8. Système de remplissage (10) comprenant au moins un dispositif de dosage (12) selon l'une quelconque des revendications précédentes et comprenant une installation CIP (14) pour le nettoyage de l'unité de remplissage au moyen d'un milieu de nettoyage dans au moins un processus de nettoyage.

9. Procédé de nettoyage d'un dispositif de dosage (12), en particulier selon l'une quelconque des revendications 1 à 7, comprenant une unité de remplissage (16) pour le transport de produits au moins liquides ou pâteux et comprenant une interface avec une installation CIP (14) pour le nettoyage de l'unité de remplissage (16) au moyen d'un milieu de nettoyage dans au moins un processus de nettoyage, au moins une grandeur caractéristique d'encrassement du milieu de nettoyage étant détectée dans le processus de nettoyage, la grandeur caractéristique d'encrassement du milieu de nettoyage étant détectée et/ou captée par un premier élément de capteur (30) d'une unité de capteur (18) du dispositif de dosage (12) disposé au niveau d'une unité de réception (50) du dispositif de dosage (12), **caractérisé en ce que** l'unité de réception (50) reçoit le milieu de nettoyage après un nettoyage d'au moins une unité de dosage (20) du dispositif de dosage (12) comprenant au moins une pompe de dosage (28) pour un dosage de produits, le milieu de nettoyage étant guidé dans une première étape de nettoyage (100) à travers une entrée d'agent de nettoyage (72) d'une unité de réservoir de produit (70) de l'unité de remplissage (16) jusqu'à une sortie de produit (26) de l'unité de dosage (20), la pompe de dosage (28) présentant la sortie de produit (26).

10. Procédé selon la revendication 9, **caractérisé en ce que** dans une deuxième étape de nettoyage (102), le milieu de nettoyage est guidé à travers une conduite d'alimentation en produit (74) de l'unité de réservoir de produit (70) jusqu'à la sortie de produit (26) de l'unité de dosage (20).

11. Procédé selon l'une quelconque des revendications 9 ou 10, **caractérisé en ce que** dans une troisième étape de nettoyage (104), le milieu de nettoyage est guidé à travers la conduite d'alimentation en produit (74) de l'unité de réservoir de produit (70) et jusqu'à une sortie arrière (29) de la pompe de dosage (28) de l'unité de dosage (20).

12. Procédé selon l'une quelconque des revendications 9 à 11, **caractérisé en ce que** dans une quatrième étape de nettoyage (106), le milieu de nettoyage est guidé à travers la conduite d'alimentation en produit (74) de l'unité de réservoir de produit (70) et jusqu'à une conduite d'équilibrage de fluide (66) de l'unité de remplissage (16), laquelle est disposée au niveau de l'unité de réservoir de produit (70).
